# EUROPEAN PATENT APPLICATION

(11) **EP 4 700 977 A1**
(43) Date of publication of application: **25.02.2026**
(21) Application number: 25197207.1
(22) Date of filing: 21.08.2025
(51) Int. Cl.: H01P 1/06, A61B 6/00, A61B 6/03, H04B 5/22, H04B 5/72

(54) **ROTARY ASSEMBLY FOR THERAPEUTIC DEVICE AND COMPUTED TOMOGRAPHY DEVICE**

(30) Priority: 21.08.2024 CN 202411157110
(71) Applicant: Shanghai United Imaging Healthcare Co., Ltd., Shanghai 201807 (CN)
(72) Inventor: LV, Tong, Shanghai, 201807 (CN); ZHANG, Wei, Shanghai, 201807 (CN); ZHOU, Zerun, Shanghai, 201807 (CN); SHI, Wei, Shanghai, 201807 (CN); CHEN, Beibei, Shanghai, 201807 (CN); ZHANG, Yang, Shanghai, 201807 (CN)
(74) Representative: Wang, Bo

(57) **Abstract**

Embodiments of the present disclosure provide a rotary assembly. The rotary assembly comprises a stator disk, a rotor disk rotationally cooperated with the stator disk, at least one transmitting antenna disposed on a mating surface of one of the stator disk or the rotor disk, and at least one receiving antenna disposed on the mating surface of the other of the stator disk or the rotor disk. The transmitting antenna and the receiving antenna are electromagnetically coupled to maintain signal integrity during relative rotation.

## Description

### CROSS-REFERENCE TO RELATED APPLICATIONS

This application claims priority to Chinese patent application No. 202411157110.9, filed on August 21, 2024, the entire contents of which are incorporated herein by reference.

### TECHNICAL FIELD

The present disclosure relates to the field of medical devices, and particularly relates to a rotary assembly for a therapeutic device and an electronic computed tomography device.

### BACKGROUND

Computed Tomography (CT) is a non-destructive testing instrument applied in the medical field. CT uses X-rays to irradiate an object in a plurality of directions, reconstructs tomographic images via a computer, and realizes internal structure analysis. A coil between a slip ring of the computed tomography device and a stationary component performs wireless energy coupling, so that the computed tomography device may realize power transmission and data transmission.

However, during the rotational interaction of the computed tomography device, carbon brushes and copper slideways will continuously rub against each other, carbon powder is generated, which will affect the normal operation of board card devices. The noise generated by the friction of the carbon brushes will also reduce the user experience. Moreover, the slip ring generally transmits power or signals via a single line. If a fault occurs at a certain position of the coil (e.g., wire breakage, etc.), the entire coil will be unable to transmit power or signals, thereby affecting the reliability of the operation of the computed tomography device. In addition, the large-sized slip ring may carry data transmission of a large number of channels, but the large-sized slip rings are difficult to manufacture.

Therefore, it is desirable to provide a rotary assembly for a therapeutic device and a computed tomography device. By adding a metal skeleton inside the large-sized slip ring to improve the rigidity. Moreover, the fabricated slip ring may reduce the amount of carbon powder generated during power and data transmission, achieve silent transmission, and ensure that power and data transmission are not affected when a fault occurs at a certain position of the coil.

### SUMMARY

One or more embodiments of the present disclosure provide a rotary assembly. The rotary assembly comprises a stator disk, a rotor disk rotationally cooperated with the stator disk, at least one transmitting antenna disposed on a mating surface of one of the stator disk or the rotor disk, and at least one receiving antenna disposed on the mating surface of the other of the stator disk or the rotor disk. The transmitting antenna and the receiving antenna are electromagnetically coupled to maintain signal integrity during relative rotation.

In some embodiments, the rotary assembly further comprises a first end-face transceiver and a second end-face transceiver. A first transmitting antenna of the first end-face transceiver is disposed on a first mating surface of the rotor disk. A first receiving antenna of the first end-face transceiver is disposed on the stator disk. A second transmitting antenna of the second end-face transceiver is disposed on a second mating surface of the stator disk. A second receiving antenna of the second end-face transceiver is disposed on the rotor disk.

In some embodiments, the transmitting antenna and the receiving antenna include microstrip lines, and the microstrip lines are concentric arcuate conductors. The conductors share a common central axis. Opposing portions of the conductors are separated by an axial air gap.

In some embodiments, the rotary assembly further comprises a receiving plate carrying the receiving antenna. The receiving plate includes an alignment mark configured to radially center the transmitting antenna relative to the receiving antenna.

In some embodiments, the transmitting antenna includes at least two microstrip lines, the at least two microstrip lines are concentric arcuate conductors spaced and concentrically arranged along a radial direction, and a length difference between two electrical paths of any two microstrip lines of the at least two microstrip lines is less than or equal to a preset threshold.

In some embodiments, at least one microstrip line comprises a compensation trace segment connected via an impedance-matching transition.

In some embodiments, the microstrip line comprises a single impedance-matching transition connected end-to-end with a single microstrip segment.

In some embodiments, the microstrip line comprises at least two impedance-matching transitions and at least two microstrip segments. Adjacent microstrip segments are interconnected through one of the at least two impedance-matching transitions.

In some embodiments, the at least two microstrip segments have identical radii. Each impedance-matching transition includes a compensation wiring. An electrical path of the microstrip line comprises the microstrip segment and the compensation wiring.

In some embodiments, a clearance between the transmitting antenna and the receiving antenna ranges from 0.5 mm to 5 mm.

In some embodiments, the rotary assembly further comprises at least one peripheral transceiver. The at least one peripheral transceiver includes a peripheral transmitting antenna disposed on a circumferential surface of one of the stator disk or the rotor disk, and a peripheral receiving antenna disposed on the other of the stator disk or the rotor disk. The peripheral transmitting antenna and the peripheral receiving antenna are radially opposed.

In some embodiments, the rotary assembly further comprises a secondary stator component, a secondary rotor component rotationally cooperated with the secondary stator component, a first wound conductor array disposed on the secondary stator component, and a second wound conductor array disposed on the secondary rotor component. The first wound conductor array comprises modular segments configured for independent parallel operation.

In some embodiments, the modular segments are detachably mounted on the secondary stator component.

In some embodiments, the secondary stator component defines a mounting aperture. The first wound conductor array is disposed on an inner peripheral wall of the mounting aperture. The secondary rotor component is rotatably disposed within the mounting aperture. The second wound conductor is disposed on an outer peripheral wall of the secondary rotor component.

In some embodiments, at least one of the stator disk or the rotor disk comprises a cast reinforcement frame embedded therein.

In some embodiments, the cast reinforcement frame forms a continuous toroidal structure.

In some embodiments, a radial span of the cast reinforcement frame occupies 5-20% of a diameter of the disk.

In some embodiments, the cast reinforcement frame includes at least one hollow portion, and the at least one hollow portion is centrally symmetric about an axis of the disk.

In some embodiments, the cast reinforcement frame comprises a plurality of detachably interconnected grid plates and cross-member. The grid plates include threaded bores configured for engagement with ejection fixtures during demolding.

One or more embodiments of the present disclosure provide a computed tomography device. The computed tomography device including a gantry and a rotary assembly mounted on the gantry.

### BRIEF DESCRIPTION OF THE DRAWINGS

The present disclosure will be further illustrated with reference to a plurality of exemplary embodiments, described in detail using the accompanying drawings. These embodiments are not limiting, and in these embodiments, the same numbering denotes the same structure, where:
FIG. 1 is a schematic structural diagram of a rotary assembly according to some embodiments of the present disclosure;
FIG. 2 is an exploded structural schematic diagram of a rotary assembly from one angle according to some embodiments of the present disclosure;
FIG. 3 is an exploded structural schematic diagram of a rotary assembly from another angle according to some embodiments of the present disclosure;
FIG. 4 is a schematic structural diagram of a first stationary component according to some embodiments of the present disclosure;
FIG. 5 is an enlarged schematic diagram of a portion A in FIG. 4;
FIG. 6 is a schematic structural diagram of a first rotary component according to some embodiments of the present disclosure;
FIG. 7 is an enlarged schematic diagram of a portion B in FIG. 6;
FIG. 8 is a schematic layout diagram of a transceiver according to some embodiments of the present disclosure;
FIG. 9 is a schematic end-face structural diagram of a rotary assembly according to some embodiments of the present disclosure;
FIG. 10 is a cross-sectional schematic diagram along line D-D in FIG. 9;
FIG. 11 is an enlarged schematic diagram of a portion E in FIG. 10;
FIG. 12 is a schematic layout diagram of a first stationary component according to some embodiments of the present disclosure;
FIG. 13 is an enlarged schematic diagram of a portion F in FIG. 12;
FIG. 14 is a schematic structural diagram of an end face transmitting antenna according to some embodiments of the present disclosure;
FIG. 15 is a schematic structural diagram of a first bend section according to some embodiments of the present disclosure;
FIG. 16 is a schematic structural diagram of a transition plate and a microstrip line segment according to some embodiments of the present disclosure;
FIG. 17 is a schematic diagram illustrating a structure of two microstrip line segments with different radii according to some embodiments of the present disclosure;
FIG. 18 is a schematic diagram illustrating a structure of a compensation wiring disposed in a transition plate according to some embodiments of the present disclosure;
FIG. 19 is a schematic diagram illustrating a structure of a second bend section according to some embodiments of the present disclosure;
FIG. 20 is a schematic diagram illustrating a structure of a peripheral transceiver according to some embodiments of the present disclosure;
FIG. 21 is a schematic diagram illustrating an enlarged view of portion C in FIG. 8;
FIG. 22 is a schematic diagram illustrating a structure of installing a first rotary disk and a first stationary disk according to some embodiments of the present disclosure;
FIG. 23 is a schematic diagram illustrating an exploded structure of the rotary assembly according to some embodiments of the present disclosure;
FIG. 24 is a schematic diagram illustrating an exploded structure of a first wound conductor array and a secondary stator component according to some embodiments of the present disclosure;
FIG. 25 is a schematic diagram illustrating a three-dimensional structure of modular segments when interconnected and separated according to some embodiments of the present disclosure;
FIG. 26 is a schematic diagram illustrating a structure of installing a secondary rotor component and a secondary stator component according to some embodiments of the present disclosure;
FIG. 27 is a schematic diagram illustrating a cast reinforcement frame according to some embodiments of the present disclosure;
FIG. 28 is a schematic diagram illustrating another cast reinforcement frame according to some embodiments of the present disclosure;
FIG. 29 is a schematic diagram illustrating yet another cast reinforcement frame according to some embodiments of the present disclosure.

### Reference signs:

1, transceiver; 11, end-face transceiver; 11-1, first end-face transceiver; 11-2, second end-face transceiver; 111, end face transmitting antenna; 111-1, first end face transmitting antenna; 111-2, second end face transmitting antenna; 1111, microstrip line; 1111a, first microstrip line; 1111b, second microstrip line; 1111-1, first bend section; 1111-2, transition plate; 1111-21, compensation wiring; 1111-21a, first compensation wiring; 1111-21b, second compensation wiring; 1111-211, second bend section; 1111- 22, termination resistor; 1111-23, reference GND; 1111-24, solder joint; 1111-3, microstrip line segment; 1111-3a, first microstrip line segment; 1111-3b, second microstrip line segment; 112, end face receiving antenna; 112-1, first end face receiving antenna; 112-2, second end face receiving antenna; 113, end face receiving plate; 1131 , end face alignment mark; 114, end face transmitting plate; 12, peripheral transceiver; 121, peripheral transmitting antenna; 1211, peripheral surface; 122, peripheral surface; 123, receiving plate; 1231, alignment mark; 124, peripheral surface transmitting plate; 13, first wound conductor array; 131, modular segments; 14, second wound conductor array; 141, second wound conductor array body; 2, first stationary component; 21, first stationary disk; 211, first locating hole; 3, first rotary component; 31, first rotary disk; 311, second locating hole; 312, notch; 4, secondary stator component; 41, second stationary disk; 411, mounting aperture; 5, secondary rotor component; 51, second rotary disk; 52, winding post; 6, cast reinforcement frame; 61, hollow portion; 62, grid plate; 621, threaded bore; 63, cross-member; M, first mating surface; N, second mating surface.

### DETAILED DESCRIPTION

In order to more clearly illustrate the technical solutions of the embodiments of the present disclosure, the accompanying drawings to be used in the description of the embodiments will be briefly described below. Obviously, the accompanying drawings in the following description are only some examples or embodiments of the present disclosure, and it is possible for those skilled in the art to apply the present disclosure to other similar scenarios based on the accompanying drawings without creative labor. Unless obviously obtained from the context or the context illustrates otherwise, the same numeral in the drawings refers to the same structure or operation.

It will be understood that the term "system," "engine," "unit," "module," and/or "block" used herein are one method to distinguish different components, elements, parts, sections, or assembly of different levels in ascending order. However, the terms may be displaced by another expression if they achieve the same purpose.

As used herein, the singular forms "a," "an," and "the" may be intended to include the plural forms as well, unless the context clearly indicates otherwise. It will be further understood that the terms "comprise", "comprises", and/or "comprising", "include", "includes" and/or "including", when used in this disclosure, specify the presence of stated features, integers, steps, operations, elements, and/or components, but do not preclude the presence or addition of one or more other features, integers, steps, operations, elements, components, and/or groups thereof.

Slip rings and stationary components in a computed tomography device are configured to achieve power transmission and data transmission. For example, a coil of the stationary component is energized with a high-frequency alternating current to generate an alternating magnetic field, and a coil of the slip ring cuts the magnetic lines of force to generate an induced current, thereby achieving power transmission. As another example, during rotation, when the peripheral antenna of the slip ring is coupled to the receiving antenna of the stationary component, image data is transmitted from the slip ring to the stationary component; when the copper slideway on the disk of the slip ring is in contact with the carbon brush on the stationary component, control data is transmitted from the stationary component to the slip ring.

During the rotational interaction, the carbon brushes and the copper slideways will continuously rub against each other, generating carbon powder. The carbon powder will affect the normal operation of the board devices; meanwhile, the noise generated by the friction of the carbon brushes will also reduce the user experience. Slip rings generally transmit power or signals via a single line. If a fault occurs at a certain position of the coil (e.g., wire breakage, etc.), the entire coil will be unable to transmit power or signals, thus affecting the reliability of the operation of mechanical equipment. The large-sized slip rings may carry data transmission of more channels, but the large-sized slip rings are difficult to manufacture.

To solve the problems existing in the power transmission process, CN112701548B discloses a power signal shaft structure of an electrical slip ring and a processing device. By coaxially arranging a conductive ring, a centering member and a mandrel, and providing an injection-molded part configured to fix the conductive ring between the conductive ring and the centering member, it achieves connection stability and service life of the product.

To solve the problems existing in the data transmission process, CN108926354A discloses a modularly supported rotary component for capacitive data links, wherein the stationary component and the slip ring are arranged as a non-contact structure, and the data transceiver module is coupled via arcuate surfaces to achieve power transmission.

The present disclosure discloses a rotary assembly for a therapeutic device and a computed tomography device, which achieves contactless data transmission through antenna coupling between transmitting antennas and receiving antennas by arranging the transmitting antennas and the receiving antennas on a stationary component and a rotary component respectively, thereby improving the stability of data transmission through the rotary assembly of a data link and reducing carbon powder pollution and noise generation during operation; meanwhile, by setting the lengths of at least a portion of the transmitting antennas to be equal, the signal transmission quality is improved and the signal transmission stability is ensured; furthermore, by arranging a plurality of independent and parallel modular segments, when a failure occurs somewhere in the coil, power transmission and data transmission are not affected; finally, by adding a metal frame inside a large-sized slip ring, the rigidity is improved and the service life of the slip ring is prolonged.

Some embodiments of the present disclosure provide a rotary assembly for a therapeutic device (hereinafter referred to as the rotary assembly).

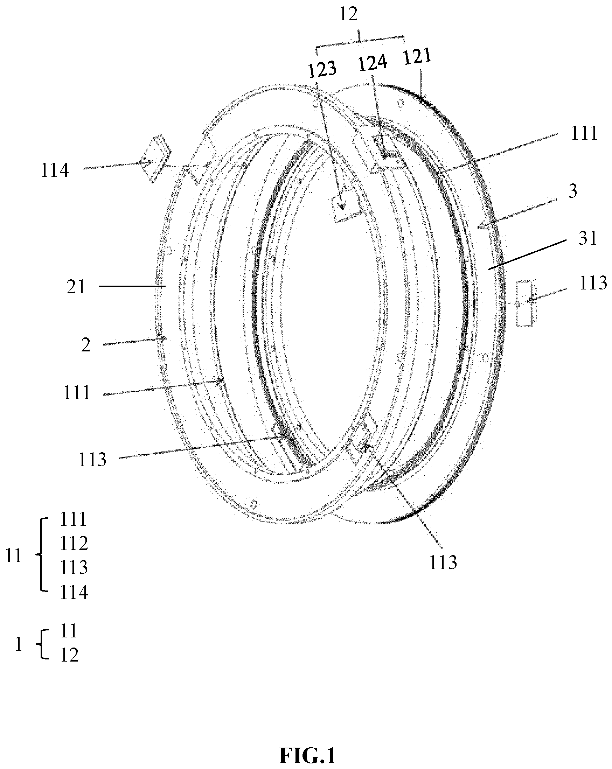

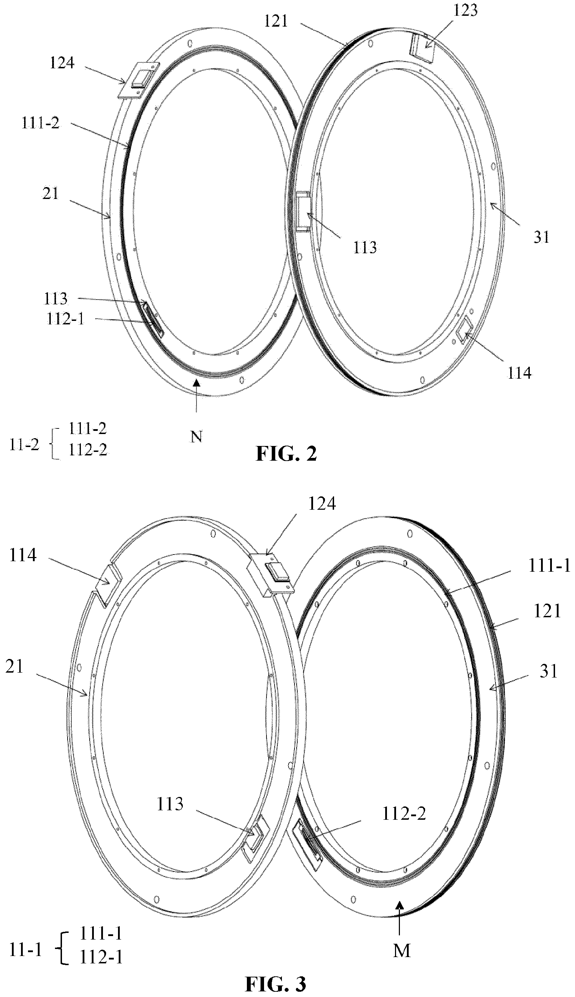

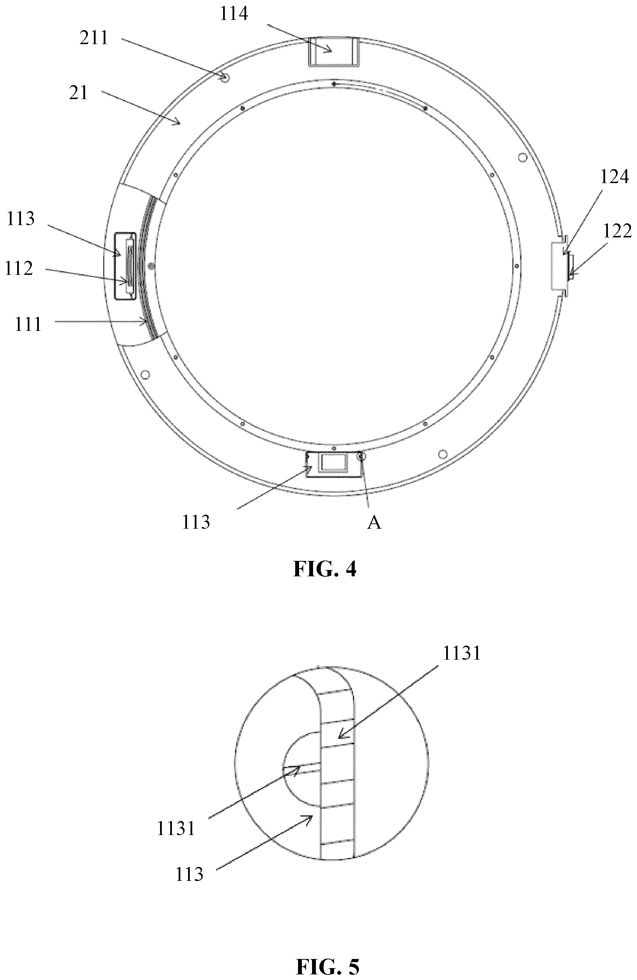

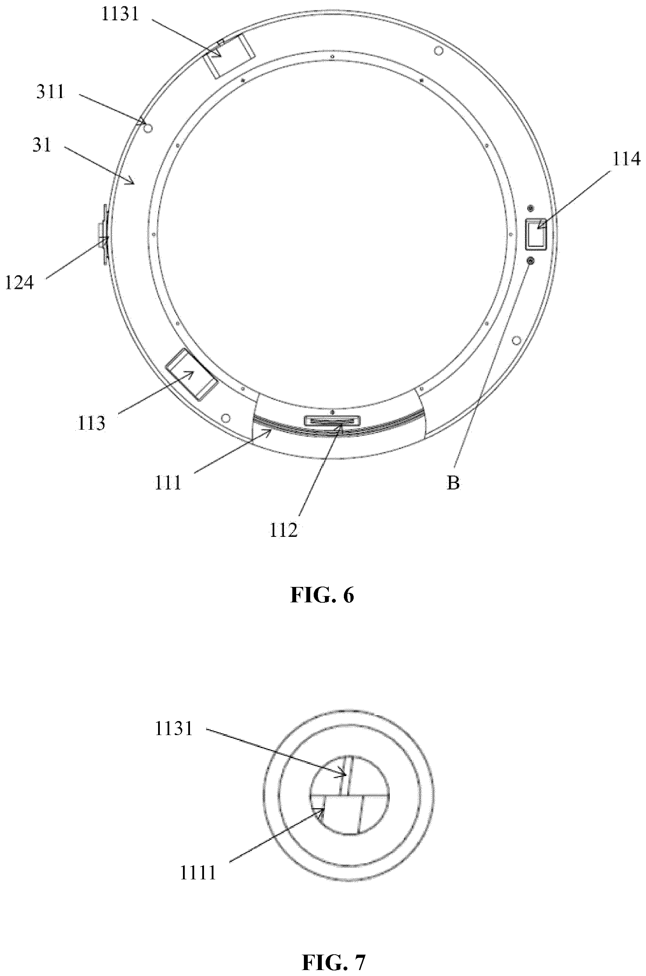

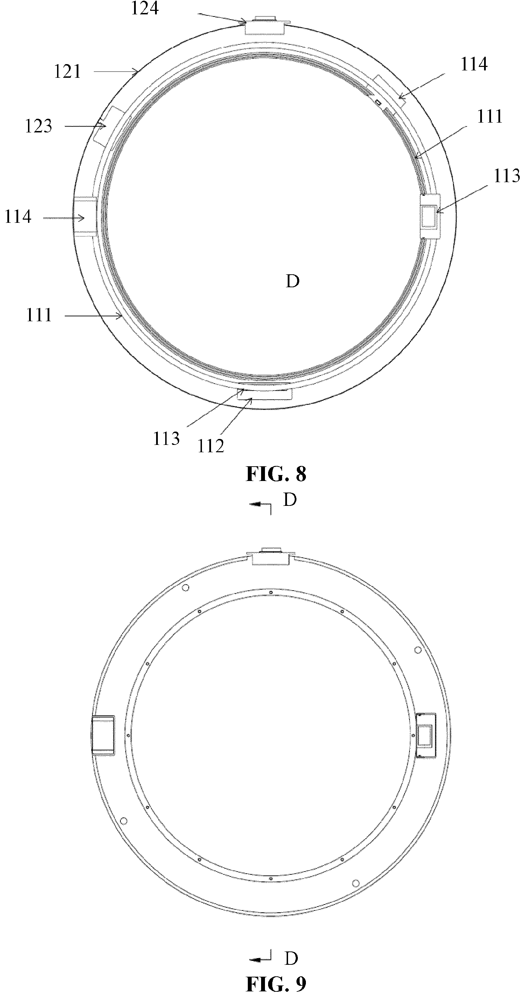

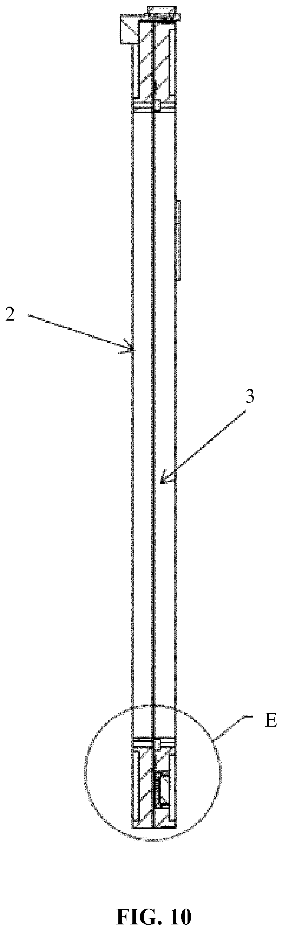

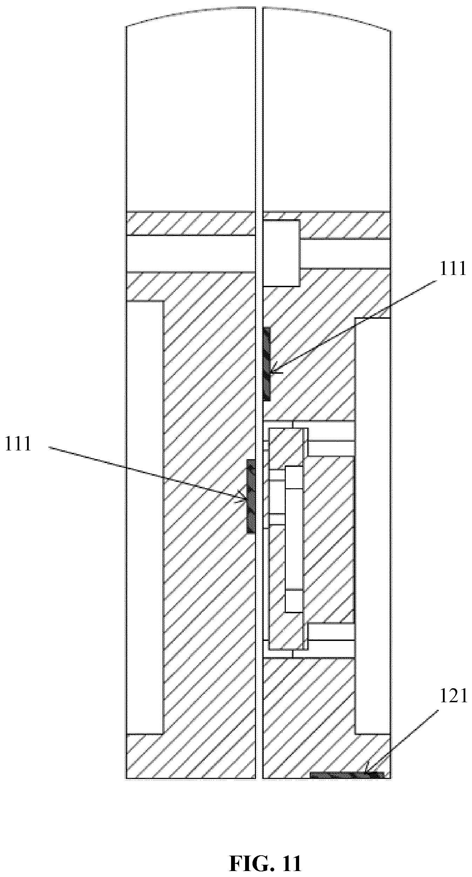

FIG. 1 is a schematic structural diagram of a rotary assembly according to some embodiments of the present disclosure; FIG. 2 is an exploded structural schematic diagram of a rotary assembly from one angle according to some embodiments of the present disclosure; FIG. 3 is an exploded structural schematic diagram of a rotary assembly from another angle according to some embodiments of the present disclosure; FIG. 4 is a schematic structural diagram of a first stationary component according to some embodiments of the present disclosure; FIG. 5 is an enlarged schematic diagram of a portion A in FIG. 4; FIG. 6 is a schematic structural diagram of a first rotary component according to some embodiments of the present disclosure; FIG. 7 is an enlarged schematic diagram of a portion B in FIG. 6; FIG. 8 is a schematic layout diagram of a transceiver according to some embodiments of the present disclosure; FIG. 9 is a schematic end-face structural diagram of a rotary assembly according to some embodiments of the present disclosure; FIG. 10 is a cross-sectional schematic diagram along line D-D in FIG. 9; FIG. 11 is an enlarged schematic diagram of a portion E in FIG. 10.

In some embodiments, the rotary assembly includes a stator disk, a rotor disk, at least one transmitting antenna, and at least one receiving antenna. The rotor disk is rotationally cooperated with the stator disk. The at least one transmitting antenna is disposed on a mating surface of one of the stator disk or the rotor disk. The at least one receiving antenna is disposed on the mating surface of the other of the stator disk or the rotor disk. The transmitting antenna and the receiving antenna are electromagnetically coupled to maintain signal integrity during relative rotation.

In some embodiments, the mating surface refers to a surface on the stator disk and the rotor disk configured for positioning the transmitting antenna and the receiving antenna. In some embodiments, the mating surface includes at least an end face. For example, the mating surface includes the end face, a circumferential surface, etc. For example, the transmitting antenna is disposed on the end face of either the stator disk or the rotor disk, and the receiving antenna is also disposed on the end face of the other one of the stator disk or the rotor disk. As another example, the transmitting antenna is disposed on the circumferential surface of either the stator disk or the rotor disk, and the receiving antenna is disposed on the circumferential surface of the other one of the stator disk or the rotor disk. As another example, the transmitting antenna is disposed on the end face of either the stator disk or the rotor disk, and the receiving antenna is disposed on the circumferential surface of the other one of the stator disk or the rotor disk.

In some embodiments, as shown in FIG. 1 - FIG. 11, the rotary assembly includes a transceiver 1, a first stationary component 2, and a first rotary component 3 rotationally cooperating with the first stationary component 2. The transceiver 1 includes at least one set of end-face transceiver 11. The at least one set of end-face transceiver 11 includes an end face transmitting antenna 111 and an end face receiving antenna 112. The first rotary component 3 includes a first rotary disk 31. The first stationary component 2 includes a first stationary disk 21. The end face transmitting antenna 111 is arranged on an end face of one of the first rotary disk 31 and the first stationary disk 21. The end face receiving antenna 112 is arranged on the other of the first rotary disk 31 and the first stationary disk 21. The end face transmitting antenna 111 and the end face receiving antenna 112 are coupled to each other to achieve data transmission.

The first stationary component 2 is a component of the rotary assembly that is configured to secure and support the first rotary component 3. In some embodiments, the first stationary component 2 remains stationary in the rotary assembly. The first stationary component 2 is also referred to as the stator disk.

The first stationary disk 21 is a main structure of the first stationary component 2. The first stationary disk 21 can be designed in a variety of shapes, such as a circle, a cogwheel, and the like. In some embodiments, a dimension of the first stationary disk 21 is determined based on demand.

The first rotary component 3 refers to a part of the rotary assembly that rotates about a central axis to adjust antenna orientation. In some embodiments, the first stationary component 2 and the first rotary component 3 are coaxially provided and form a rotational fit in various ways. For example, the first rotary component 3 forms a rotational fit directly with the first stationary component 2. As another example, an intermediate member (e.g., a bearing) is provided between the first rotary component 3 and the first stationary component 2 to form a rotational fit.

The term "rotational fit" refers to that, in terms of mechanical assembly, the first rotary disk 31 and the first stationary disk 21 are capable of relative rotation.

The first rotary disk 31 is a main structure of the first rotary component 3. In some embodiments, the first rotary disk 31 is coaxially provided with the first stationary disk 21. The first rotary disk 31 is also referred to as the rotor disk.

In some embodiments, alignment pin holes are pre-determined on the first rotary disk 31 and/or the first stationary disk 21, and the first rotary disk 31 and the first stationary disk 21 are matched mounted via the alignment pin holes.

In some embodiments, a shape and a dimension of the first rotary disk 31 match a shape and a dimension of the first stationary disk 21, as shown in FIG. 6. In some embodiments, there is a gap between the first rotary disk 31 and the first stationary disk 21 to enable non-contact interaction.

The transceiver 1 is configured to enable data transmission. In some embodiments, the transceiver 1 may be arranged at an end face, a peripheral surface, or other location of the first stationary component 2 and the first rotary component 3 to transmit a plurality of types of data (e.g., control data, image data, etc.) between the first stationary component 2 and the first rotary component 3. For example, the control data is transmitted from the first stationary component 2 to the first rotary component 3 via the transceiver 1. The image data is transmitted from the first rotary component 3 to the first stationary component 2 via the transceiver 1.

The transceiver includes a transmitting antenna and a receiving antenna. The transmitting antenna is configured to send the control data and/or the image data between the first stationary component 2 and the first rotary component 3, and the receiving antenna is configured to receive the control data and/or the image data sent by the corresponding transmitting antenna.

In some embodiments, the transmitting antenna may be disposed on an end face, a peripheral surface, or other location of the first stationary component 2. For example, when the transmitting antenna is provided on an end face of the first stationary disk 21, the transmitting antenna is provided on an end face of the first stationary disk 21 facing the first rotary disk 31. As another example, when the transmitting antenna is provided at an end face of the first rotary disk 31, the transmitting antenna is provided at an end face of the first rotary disk 31 facing the first stationary disk 21.

In some embodiments, the transmitting antenna and the receiving antenna may be made of a material having good electrical conductivity and electromagnetic properties, such as copper, silver, or the like.

In some embodiments, a curvature of the transmitting antenna and the receiving antenna matches a curvature of a disk (the first stationary disk 21 or the first rotary disk 31) on which the transmitting antenna or the receiving antenna is arranged, and the transmitting antenna and the receiving antenna are coaxially arranged with that disk. For example, the transmitting antenna and the receiving antenna may be antennas in a shape of a circle that surrounds a disk body on which the transmitting antenna and the receiving antenna are provided. As another example, the transmitting antenna and the receiving antenna may also be antennas with a plurality of arc-shaped segments, which are evenly distributed around the disk on which the transmitting antenna and the receiving antenna are disposed.

In some embodiments, the transmitting antenna and the receiving antenna may each comprise one or more loops of antennas.

In some embodiments, the transceiver includes a plurality of transmitting antennas. In some embodiments, at least a portion of the plurality of transmitting antennas are equal in length. For example, transmitting antennas in a transceiver have equal lengths. For instance, a transceiver includes two end face transmitting antennas, lengths of the two end face transmitting antennas are equal. As another example, the rotary assembly includes a plurality of transceivers. Some transceivers among the plurality of transceivers have transmitting antennas of an equal length. As another example, the transmitting antennas of the plurality of transceivers are all of an equal length.

Notably, the transmitting antenna may be arranged at any one or more of the end face or the peripheral surface of the first stationary component, and the end face or the peripheral surface of the first rotary component. The receiving antenna may be correspondingly arranged at a position where signals may be received. That is, the position of the transmitting antenna can be set according to the signal transmission demand of the transceiver 1.

It will be appreciated that the transceiver for transmitting the control data and the image data may be arranged on the end face of the disk (such as an end-face transceiver, including an end face transmitting antenna and an end face receiving antenna), on the peripheral surface (such as a peripheral transceiver, including a peripheral transmitting antenna and a peripheral receiving antenna), and other positions where there is no obstruction between the transmitting antenna and the corresponding receiving antenna, so as to meet the signal transmission requirements.

The end-face transceiver 11 is a transceiver provided on the end faces of the first stationary component 2 and the first rotary component 3. The term "end face" refers to a plane on an object perpendicular to its axis. In some embodiments, the end-face transceiver 11 may be one or more, which may be determined based on the actual amount or type of data to be transmitted. Taking an example of only one end-face transceiver 11, the end-face transceiver 11 may transmit the control data and the image data in both directions. Taking a plurality of end-face transceivers 11 as an example, a portion of the end-face transceivers 11 are configured to transmit the control data, and another portion of the end-face transceivers 11 are configured to transmit image data.

The end face transmitting antenna 111 is a transmitting antenna provided at an end face of the first stationary component 2 and/or the first rotary component 3, and the end face receiving antenna 112 is a receiving antenna configured to couple with the end face transmitting antenna 111 in correspondence with each other. It should be noted that the end face receiving antenna 112 is not limited to being provided at an end face of the first stationary component 2 and/or the first rotary component 3.

The end face transmitting antenna 111 and the end face receiving antenna 112 are coupled in correspondence with each other in that the end face transmitting antenna 111 and the end face receiving antenna 112 are coupled with each other by the interaction of the electromagnetic field, so that a signal can be transmitted between the end face transmitting antenna 111 and the end face receiving antenna 112.

For example, when a count of the end-face transceiver 11 is only one, the end face transmitting antenna 111 and the end face receiving antenna 112 are coupled with each other to enable the bi-directional transmission of the control data and the image data. As another example, when a count of the end-face transceiver 11 is at least two, the end face transmitting antennas 111 and the end face receiving antennas 112 of the at least one end-face transceiver 11 are coupled with each other to realize the transmission of the control data; at least one end-face transceiver 11 has the end face transmitting antenna 111 and the end face receiving antenna 112 coupled to each other to enable the transmission of the image data.

In some embodiments, for a same end-face transceiver 11, the end face transmitting antenna 111 may be provided on an end face of the first stationary disk 21 or the first rotary disk 31 (e.g., an end face of the first stationary disk 21 facing the first rotary disk 31 or an end face of the first rotary disk 31 facing the first stationary disk 21). The end face receiving antenna 112 is disposed on an unobstructed position of the first stationary disk 21 or the first rotary disk 31 on which the end face transmitting antenna 111 is not disposed. The end face transmitting antenna 111 and the end face receiving antenna 112 are relatively spaced apart and correspondingly arranged (e.g., the end face transmitting antenna 111 and the end face receiving antenna 112 do not contact each other, and neither is disposed on a same disk), so as to form capacitive coupling. An unobstructed position is a position such that there is no obstruction between the end face receiving antenna 112 and the end face transmitting antenna 111 by other structures. That is to say, the end face receiving antenna 112 may be provided on the end face of a disk (e.g., the first stationary disk 21 or the first rotary disk 31, also referred to as "disk"), or it may be provided on the peripheral surface of the disk or at other positions that can form a capacitive coupling with the end face transmitting antenna 111.

In some embodiments, for a plurality of different end-face transceivers 11, the end face transmitting antennas 111 of each end-face transceiver 11 may all be arranged on the end face of the first stationary disk 21 or the first rotary disk 31. The end face receiving antennas 112 are arranged in unobstructed positions on the first stationary disk 21 or the first rotary disk 31 where the end face transmitting antennas 111 are not arranged. Alternatively, some of the end face transmitting antennas 111 of each end-face transceiver 11 are arranged on the end face of the first stationary disk 21. The other part is arranged on the end face of the first rotary disk 31. The end face receiving antennas 112 are arranged in unobstructed positions on the first stationary disk 21 or the first rotary disk 31 where the end face transmitting antennas 111 are not arranged.

It should be noted that the end face receiving antenna 112 is provided at a spaced apart position from the end face transmitting antenna 111, which also refers to an existence of a gap between the first rotary component 3 and the first stationary component 2 and is not a pasting contact, that is to say that the rotary assembly is non-contact.

In some embodiments, the end face transmitting antenna 111 and the end face receiving antenna 112 are provided on the disk (the first stationary disk 21 and/or the first rotary disk 31) in a variety of ways. For example, the disk may be provided with an inner cavity to embed the end face transmitting antenna 111 or the end face receiving antenna 112 in the inner cavity. As another example, the end face transmitting antenna 111 and the end face receiving antenna 112 may also be provided on the disk by a snap connection, a bonding, etc.

In some embodiments of the present disclosure, by arranging transmitting antennas and receiving antennas on the first stationary disk and the first rotary disk respectively, the transmitting antennas and receiving antennas are coupled with each other to realize data transmission between the first stationary disk and the first rotary disk. This enables the control data and the image data between the first stationary disk and the first rotary disk to be transmitted via antenna coupling, in a non-contact wireless manner. This not only achieves silent and pollution-free transmission of a plurality of pieces of data, but also improves the stability of data transmission between the first stationary disk and the first rotary disk, thereby enhancing the operational stability of the rotary assembly in the entire data link. At the same time, by setting the lengths of at least a portion of the transmitting antennas to be equal, the quality of the data transmission is improved and the stability of the data transmission is improved. In addition, when a plurality of sets of antennas are arranged on the first stationary disk and/or the first rotary disk, the peripheral surface may only be distributed along an axial direction of the disk, which causes the first stationary disk and/or the first rotary disk to become thicker, thereby affecting the distance between transmitting antennas and receiving antennas and in turn affecting the stability of data transmission. But end-face antennas are distributed along the radial direction of the first stationary disk and/or the first rotary disk, which does not make the first stationary disk and/or the first rotary disk thicker, thus being more conducive to ensuring the stability of data transmission.

It is to be understood that the transceiver 1 may include the plurality of end-face transceivers 11. When the plurality of end face transmitting antennas 111 of the plurality of end-face transceivers 11 are arranged on the end face of the first stationary disk 21 and/or the first rotary disk 31, these end face transmitting antennas 111 may be distributed along the radial direction of the first stationary disk 21 and/or the first rotary disk 31.

In some embodiments, the rotary assembly further includes a first end-face transceiver and a second end-face transceiver. A first transmitting antenna of the first end-face transceiver is disposed on a first mating surface of the rotor disk. A first receiving antenna of the first end-face transceiver is disposed on the stator disk. A second transmitting antenna of the second end-face transceiver is disposed on a second mating surface of the stator disk. A second receiving antenna of the second end-face transceiver is disposed on the rotor disk.

In some embodiments, as shown in FIG. 1 - FIG. 11, the transceiver 1 includes two end-face transceivers 11. The end face transmitting antenna 111 of one of the two end-face transceivers 11 is arranged on a first mating surface M. The first mating surface M is the end face of the first rotary component 3 facing the first stationary component 2, and the end face receiving antenna 112 is arranged on the first stationary component 2. The end face transmitting antenna 111 of the other of the two end-face transceivers 11 is arranged on a second mating surface N. The second mating surface N is the end face of first stationary component 2 facing the first rotary component 3, and the end face receiving antenna 112 is arranged on the first rotary component 3.

In some embodiments, as shown in FIG. 2 - FIG. 3, the two end-face transceivers 11 include the first end-face transceiver 11-1 and the second end-face transceiver 11-2. The first end face transmitting antenna 111-1 of the first end-face transceiver 11-1 is arranged on the first mating surface M of the first rotary disk 31, and the first end face receiving antenna 112-1 is arranged in an unobstructed position on the first stationary disk 21. The second end face transmitting antenna 111-2 of the second end-face transceiver 11-2 is arranged on the second end face N of the first stationary disk 21, and the second end face receiving antenna 112-2 is arranged in an unobstructed position on the first rotary disk 31. The first end face transmitting antenna 111-1 is also referred to as a first transmitting antenna. The first end face receiving antenna 112-1 is also referred to as a first receiving antenna. The second end face transmitting antenna 111-2 is also referred to as a second transmitting antenna. The second end face receiving antenna 112-2 is also referred to as a second receiving antenna.

In some embodiments, the first end face transmitting antenna 111-1 and the first end face receiving antenna 112-1 are oppositely spaced and correspondingly arranged to form a capacitively coupled antenna. The second end face transmitting antenna 111-2 and the second end face receiving antenna 112-2 are oppositely spaced and correspondingly arranged to form a capacitively coupled antenna.

In some embodiments, one of the two end-face transceivers 11 is configured to transmit the control data, and the other end-face transceiver 11 is configured to transmit the image data. For example, the end face transmitting antenna 111 is arranged on the first stationary disk 21, and the end face receiving antenna 112 is arranged on the end-face transceiver 11 of the first rotary disk 31 to transmit the control data from the first stationary component 2 to the first rotary component 3. The end face transmitting antenna 111 is arranged on the first rotary disk 31, and the end face receiving antenna 112 is arranged on the end-face transceiver 11 of the first stationary disk 21 to transmit the image data from the first rotary component 3 to the first stationary component 2.

In some embodiments of the present disclosure, two transceivers are provided, which can respectively meet the data transmission requirements of the stationary component and the rotary component. In addition, the end face transmitting antenna is arranged on the end face of the first stationary disk or the first rotary disk, and the end face receiving antenna is arranged in an unobstructed position. This makes the positional layout of the end face transmitting antenna and the end face receiving antenna simple, facilitating antenna installation.

In some embodiments, a clearance between the transmitting antenna and the receiving antenna ranges from 0.5 mm to 5 mm. In some embodiments, the clearance between the transmitting antenna and the receiving antenna is a distance between the transmitting antenna and the receiving antenna along the axial direction of the first stationary disk 21.

In some embodiments, a distance between the end face transmitting antenna 111 and the end face receiving antenna 112 along the axial direction of the first stationary disk 21 ranges from 0.5 mm to 5 mm.

In some embodiments, a distance between the end face transmitting antenna 111 and the end face receiving antenna 112 along the axial direction of the first stationary disk 21 ranges from 0.5mm~1.5mm. In some embodiments, a distance between the end face transmitting antenna 111 and the end face receiving antenna 112 along the axial direction of the first stationary disk 21 ranges from 1.5mm~5mm. In some embodiments, a distance between the end face transmitting antenna 111 and the end face receiving antenna 112 along the axial direction of the first stationary disk 21 ranges from 0.3mm~5.5mm.

In some embodiments of the present disclosure, if the distance between the end face transmitting antenna and the end face receiving antenna is too close (e.g., 0.2mm-0.3mm), which will cause the first rotary component and the first stationary component to be too close, and structural interference is likely to occur during rotation. While if the distance between the end face transmitting antenna and the end face receiving antenna is too far, which will cause unstable signal reception and signal leakage. Thus, limiting the distance between the end face transmitting antenna and the end face receiving antenna along the axial direction of the first stationary disk may effectively improve the stability and quality of signal transmission.

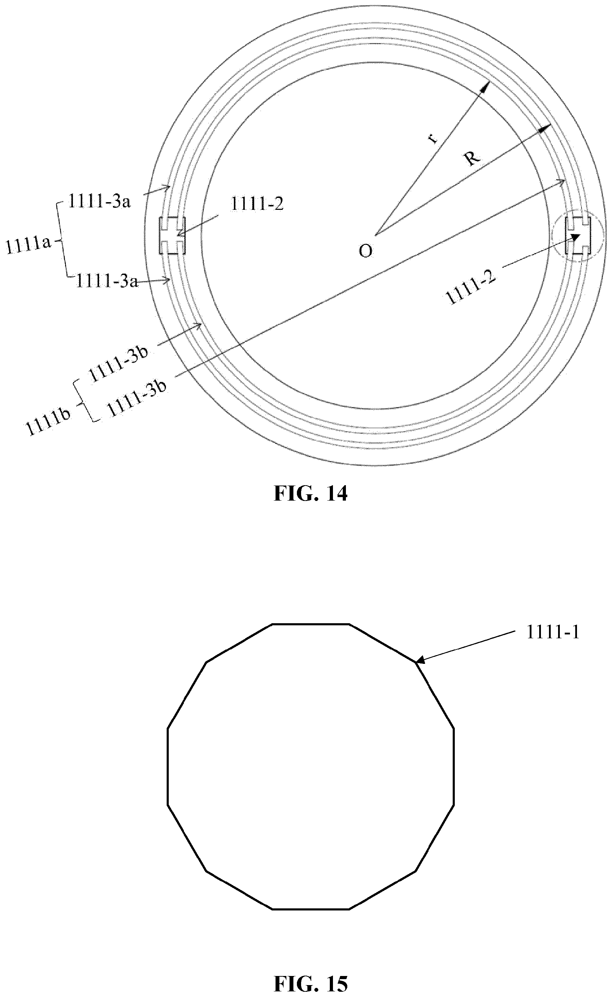

In some embodiments, the transmitting antenna and the receiving antenna include microstrip lines. The microstrip lines are concentric arcuate conductors. The conductors share a common central axis. Opposing portions of the conductors are separated by an axial air gap. The term "axial" refers to axial direction of the first stationary disk 21. For example, the transmitting antenna and the receiving antenna are arc-shaped, and the centers of the transmitting antenna and the receiving antenna are collinear along the axis of the first stationary disk 21. Moreover, the transmitting antenna and the receiving antenna are arranged relative to each other along the axis of the first stationary disk 21 and are spaced apart (that is, there is a certain gap between the transmitting antenna and the receiving antenna). More descriptions of the microstrip lines may be found in FIG. 14 to FIG. 17 and related descriptions thereof.

In some embodiments, both the end face receiving antenna 112 and the end face transmitting antenna 111 are arc-shaped. The end face transmitting antenna 111 is coaxially arranged with the end face receiving antenna 112, and the end face receiving antenna 112 is axially opposite to at least part of the end face transmitting antenna 111 along the first stationary disk 21.

In some embodiments, a curvature of an arc formed by the end face receiving antenna 112 and the end face transmitting antenna 111 matches a curvature of the disk (e.g., the first stationary disk 21 or the first rotary disk 31) on which they are located. For example, the curvature of the end face transmitting antenna 111 lies between a maximum curvature (e.g., a curvature of an outer contour line) and a minimum curvature (e.g., a curvature of an inner contour line) of the first stationary disk 21.

In some embodiments, as shown in FIG. 1 - FIG. 4, the end face transmitting antenna 111 is arranged on the end face of the first rotary disk 31. The end face transmitting antenna 111 is arc-shaped, and the end face receiving antenna 112 is also arc-shaped. A center of the end face transmitting antenna 111 and a center of the end face receiving antenna 112 are collinear along the axial direction of the first stationary disk 21. The end face transmitting antenna 111 and the end face receiving antenna 112 are axially opposite and spaced apart along the first stationary disk 21. A center of a circle is a center within a cylindrical surface in which the end face transmitting antenna 111 or the end face receiving antenna 112 is located.

In some embodiments, the coaxial arrangement of the end face transmitting antenna 111 and the end face receiving antenna 112 may be understood as follows: the center of the end face transmitting antenna 111 and the center of the end face receiving antenna 112 are collinear along the axial direction of the first stationary disk 21.

In some embodiments of the present disclosure, the arc-shaped end face receiving antenna and the end face transmitting antenna are arranged coaxially and axially opposite to each other. This allows the end face transmitting antenna and the end face receiving antenna to lie within a same circumferential plane of the first stationary disk, which is a plane passing through the end face transmitting antenna and parallel to the axial direction of the first stationary disk. Consequently, the data transmission occurs between the end face transmitting antenna and the end face receiving antenna within this circumferential plane, enabling well matching and mapping between the antennas (e.g., the transmitting antenna and the receiving antenna). This in turn ensures stable data transmission and consistent test results. Additionally, since the transmitting antenna and the receiving antenna are coaxially and coplanarly arranged, the installation and integration of the transmitting antenna and the receiving antenna are easy.

In some embodiments, a central angle of the end face receiving antenna 112 is less than 360°.

It is worth stating that the central angle of the end face transmitting antenna 111 is typically 360° to achieve omni-directional radiation and ensure uniform signal coverage in all directions. When the end face receiving antenna 112 is a directional radio frequency identification, or a magnetic resonance receiving end, the end face receiving antenna 112 only needs to be aligned with a direction of a transmitting source. A narrow-angle design can improve energy reception efficiency, reduce interference, and optimize signal strength, so the central angle of the end face receiving antenna 112 is less than 360° (as shown in FIG. 2 - FIG. 4 and FIG. 6). For example, the central angle of the end face receiving antenna 112 may be 15°, 30°, 45°, and the like.

In one embodiment, see FIG. 1 and FIG. 6, the end face transmitting antenna 111 extends along a circumferential direction of the first stationary disk 21 to form a closed-loop antenna, and the end face receiving antenna 112 is an arc antenna extending along the circumferential direction of the first stationary disk 21. When the first rotary disk 31 rotates relative to the first stationary disk 21, the end face receiving antenna 112 sequentially axially opposes different circumferential parts of the end face transmitting antenna 111 to form capacitive coupling, thus enabling the data transmission.

In some embodiments of the present disclosure, the arc-segment-shaped end face receiving antenna can achieve antenna miniaturization while ensuring a wide impedance bandwidth and high transmission power. This design reduces the size of the antenna and weight while maintaining standing wave flatness.

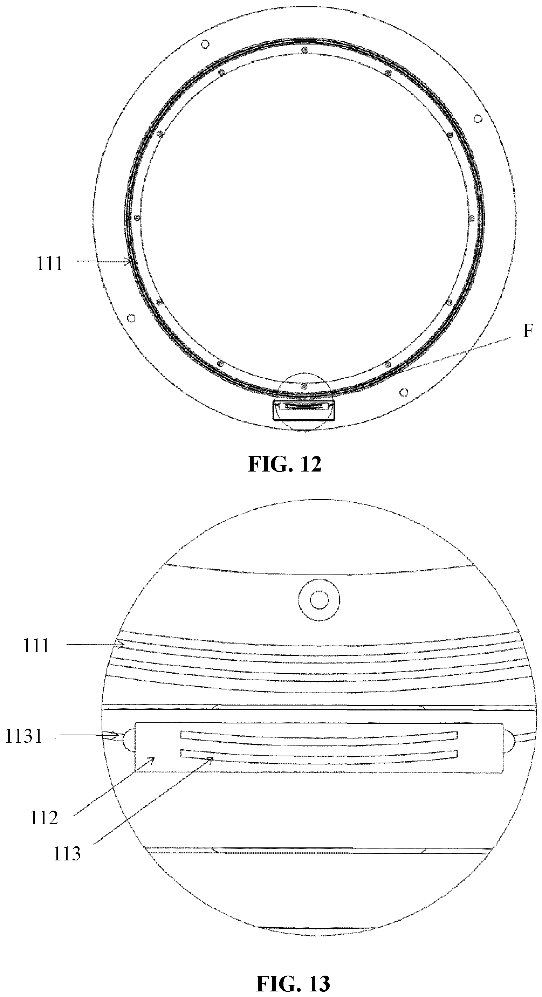

FIG. 12 is a schematic layout diagram of a first stationary component according to some embodiments of the present disclosure; FIG. 13 is an enlarged schematic diagram of a portion F in FIG. 12.

In some embodiments, the rotary assembly further includes a receiving plate carrying the receiving antenna. The receiving plate includes an alignment mark configured to radially center the transmitting antenna relative to the receiving antenna. The term "radially" refers to a radial direction of the first stationary disk 21. The receiving plate includes an end-face receiving plate and a peripheral surface receiving plate. More descriptions regarding the receiving plate can be found below.

In some embodiments, as shown in FIG. 1 - FIG. 8, FIG. 12 - FIG. 13, the end-face transceiver 11 further includes an end face receiving plate 113. The end face receiving antenna 112 is arranged on the end face receiving plate 113 and communicatively connected to the end face receiving plate 113. The end face receiving plate 113 is provided with an end face alignment mark 1131, and the end face alignment mark 1131 is configured to assist in a radial centering alignment of the end face transmitting antenna 111 and the end face receiving antenna 112 along the first stationary disk 21.

In some embodiments, as shown in FIG. 1 - FIG. 8, the end-face transceiver 11 is further provided with an end face transmitting plate 114. The end face transmitting antenna 111 is arranged on the end face transmitting plate 114 and communicatively connected to the end face transmitting plate 114. The end face transmitting plate 114 is provided with an end face alignment mark 1131, and the end face alignment mark 1131 is configured to assist in the radial centering arrangement of the end face transmitting antenna 111 and the end face receiving antenna 112 along the radial direction of the first rotary disk 31.

The term "radial centering" means that along the radial direction of the first stationary disk 21, a central plane of the end face transmitting antenna 111 coincides with a central plane of the end face receiving antenna 112.

The end face receiving plate 113 may be configured to support the end face receiving antenna 112 and transmit the control data and/or the image data received by the end face receiving antenna 112 to an external device. The end face transmitting plate 114 may be configured to support the end face transmitting antenna 111 and receive the control data and/or the image data sent by an external device for transmission to the end face transmitting antenna 111.

In some embodiments, the end face receiving plate 113 is disposed on the disk where the end face receiving antenna 112 is located, and the end face transmitting plate 114 is disposed on the disk where the end face transmitting antenna 111 is located.

For example, take the case that the end face transmitting antenna 111 is located on the first stationary component 2, and the end face receiving antenna 112 is located on the first rotary component 3 as an example, the end face receiving plate 113 is on the first rotary disk 31 at a position close to the end face transmitting antenna 111. There is no blocking structure between the end face receiving plate 113 and the end face transmitting antenna 111 to receive data (e.g., the control data) sent by the end face transmitting antenna 111.

As another example, taking the case that the end face receiving antenna 112 is disposed on the first stationary component 2 and the end face transmitting antenna 111 is disposed on the first rotary component 3 as an example, the end face transmitting plate 114 is disposed on the first stationary disk 21 at a position close to the end face receiving antenna 112. There is no blocking structure between the end face transmitting plate 114 and the end face receiving antenna 112 to receive data (e.g., the image data) sent by the end face transmitting antenna 111.

In some embodiments, as shown in FIG. 1, the end face receiving plate 113 is disposed on the first rotary disk 31, with the end face receiving antenna 112 mounted thereon and communicatively connected to the end face receiving plate 113. The end face transmitting plate 114 is disposed on the first stationary disk 21, with the end face transmitting antenna 111 mounted thereon and communicatively connected to the end face transmitting plate 114. The computer sends the control data to the end face transmitting antenna 111 via the end face transmitting plate 114. The end face transmitting antenna 111 and the end face receiving antenna 112 transfer data through capacitive coupling. Then the end face receiving antenna 112 sends the received control data to the end face receiving plate 113. The end face receiving plate 113 then sends the control data to the radiation source, detector, or the like.

In some embodiments, the end face receiving plate 113 and the end face transmitting plate 114 may be one or more; the plurality of end face receiving plates 113 and end face transmitting plates 114 are spaced apart along the circumferential direction of the disk on which the end face receiving plates 113 and the end face transmitting plates 114 are located. The end face receiving plate 113 and the end face transmitting plate 114 may be provided on the disk by welding or the like.

In some embodiments, both ends of the end face receiving antenna 112 may be connected to the end face receiving plate 113 by means of soldering, plugging, etc., thereby establishing a communicative connection with the end face receiving plate 113. Both ends of the end face transmitting antenna 111 may be connected to the end face transmitting plate 114 by means of soldering, plugging, or the like, thereby establishing a communicative connection with the end face transmitting plate 114.

The end face alignment mark 1131 is configured to assist in the radial centering alignment of the end face transmitting antenna 111 and the end face receiving antenna 112 along the first rotary disk 31. For example, the end face alignment mark 1131 may be an engraved visual marker line.

In some embodiments, see FIG. 4 and FIG. 6, the first stationary disk 21 is formed with a first locating hole 211 running axially therethrough, and the first rotary disk 31 is formed with a second locating hole 311 running axially therethrough. During assembly, the first locating hole 211 is aligned axially opposite the second locating hole 311 on the first stationary disk 21. Then, positioning pins are simultaneously inserted through both the first locating hole 211 and the second locating hole 311. The first stationary disk 21 and the first rotary disk 31 are both located in the radial and circumferential directions relative to the axis of the first stationary disk 21. Consequently, the end face transmitting antenna 111 and the end face receiving antenna 112 are precisely radially aligned.

Optionally, there may be a plurality of first locating holes 211. The plurality of first locating holes 211 are spaced apart along the circumferential direction of the first stationary disk 21. There may also be a plurality of second locating holes 311. The plurality of second locating holes 311 are spaced apart along the circumference of the first rotary disk 31. In this way, the plurality of first locating holes 211 and the plurality of second locating holes 311 may be selected nearby for positioning according to the actual installation positions of the plurality of first locating holes 211 and the plurality of second locating holes 311.

In some embodiments of this disclosure, a receiving plate and an alignment mark are provided. When assembling, a staff can use the alignment mark to ensure that the transmitting antenna and receiving antenna are aligned. This ensures stable transmission of the control data.

FIG. 14 is a schematic structural diagram of an end face transmitting antenna according to some embodiments of the present disclosure.

In some embodiments, the transmitting antenna includes at least two microstrip lines 1111, as shown in FIG. 14. In some embodiments, the at least two microstrip lines 1111 are concentric arcuate conductors spaced and concentrically arranged along a radial direction. A length difference between two electrical paths of any two microstrip lines 1111 of the at least two microstrip lines 1111 is less than or equal to a preset threshold.

A microstrip line 1111 refers to a data line within the end face transmitting antenna 111 that is configured for data transmission. The end face transmitting antenna 111 may include at least two microstrip lines 1111. The specific quantity of the at least two microstrip lines 1111 may be determined according to actual requirements. In some embodiments, the plurality of microstrip lines 1111 included in the same end face transmitting antenna 111 may be configured to transmit a same type of data (e.g., the control data or the image data).

Notably, for the plurality of different end face transmitting antennas 111, the plurality of microstrip lines 1111 included in each end face transmitting antenna 111 may be the same or different, and the lengths of the microstrip lines 1111 included in each end face transmitting antenna 111 may also be the same or different.

The term "concentrically and spaced apart in the radial direction" refers to that the plurality of microstrip lines 1111 with a same center and different radii are arranged spaced apart in the radial direction.

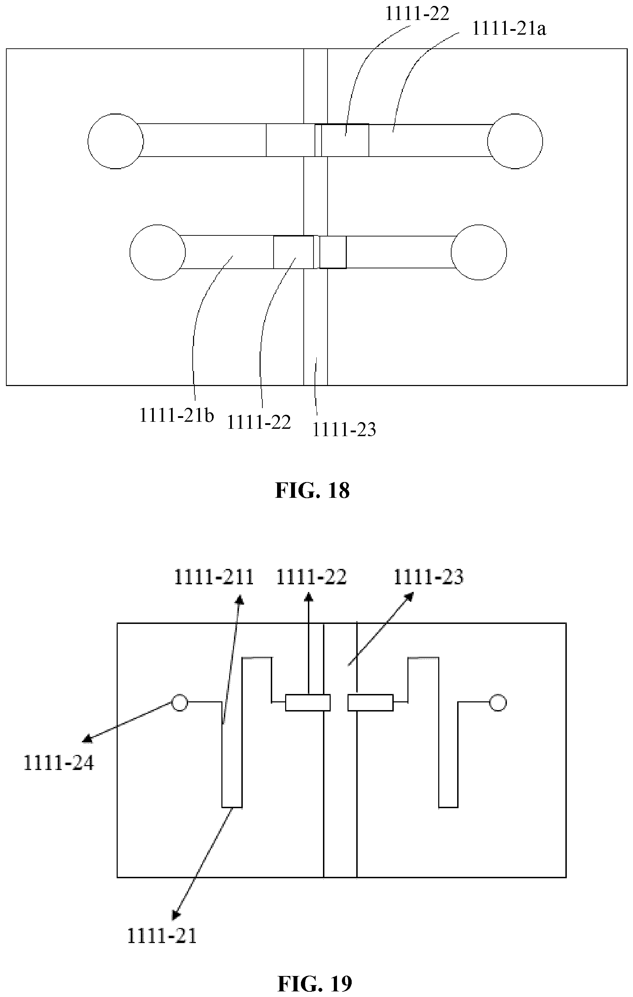

A length of an electrical path of a microstrip line refers to a total length of a current path of the microstrip line. For example, the electrical length of the microstrip line is a physical length of the microstrip line itself. As another example, when a compensation wiring is provided, the electrical length of the microstrip line is a sum of lengths of the microstrip line and the compensation wiring. For more on the compensation wiring, see FIG.18 - FIG.19 and their related descriptions.

Merely by way of example, as shown in FIG. 14, taking the end face transmitting antenna 111 including two microstrip lines 1111 as an example, the first microstrip line 1111a forms an arc with a radius R around a center O, and the second microstrip line 1111b forms an arc-shaped structure with a radius r around the center O. A spacing distance between the first microstrip line 1111a and the second microstrip line 1111b is a difference between R and r, and a length difference between two electrical paths of the first microstrip line 1111a and the second microstrip line 1111b is a difference in circumferences of the two microstrip lines' electrical paths. The difference in the circumference needs to be less than or equal to a preset threshold.

In some embodiments, the preset threshold may be set based on experience. Preferably, the preset threshold may be 0. In this case, the electrical length difference between any two microstrip lines 1111 is 0; that is, the electrical length difference between any two microstrip lines 1111 is the same.

In practical applications, transmitting antennas typically use differential pairs for signal transmission. Compared with a single signal, differential pair signals have advantages such as strong anti-interference capability, effective suppression of electromagnetic interference, and accurate timing positioning. Differential pairs need to do equal length winding and impedance control to ensure signal quality. In some embodiments, each of the transmitting antenna and the receiving antenna includes a microstrip line 1111, and the transmitting antenna includes at least two microstrip lines 1111. If the electrical lengths of the at least two microstrip lines 1111 are unequal, this will lead to different resistances among the plurality of microstrip lines 1111. This in turn will cause different time delay differences between the two signals, resulting in a phase shift at a receiving end, which further affects the integrity of the transmitted data (e.g., cause packet loss in data links, etc.). Therefore, the length of the electrical paths in the plurality of different microstrip lines 1111 should be controlled to maintain signal phase coherence. The transmitting antenna and corresponding receiving antenna are electromagnetically coupled via at least one of: capacitive field interaction, inductive field interaction, or radiative field interaction in the microwave to terahertz spectrum. The coupling mechanism encompasses capacitive coupling at lower frequencies (<1 GHz) and radiative coupling in microwave-to-terahertz bands (300 MHz-3 THz) for high-speed data transmission.

In some embodiments of this disclosure, arranging the plurality of microstrip lines on the end face of the disk (e.g., the first stationary disk 21 or the first rotary disk 31) not only avoids increasing the thickness of the disk-thus preventing an increase in the distance between the end face receiving antenna and the end face transmitting antenna, and ensuring the stability of signal transmission-but also, by using these microstrip lines for signal transmission via differential pairs, advantages over a single signal, such as strong anti-interference capability, effective suppression of electromagnetic interference, and accurate timing positioning, are offered. This effectively ensures the quality of signal transmission.

It is understandable that when the spacing distance between any two microstrip lines 1111 is too large, the electrical length difference between the microstrip lines 1111 will be too significant, which will cause impedance mismatch, be unfavorable to uniform data transmission, and thus structures for compensating electrical length (such as the first bend section 1111-1 or the compensation wiring 1111-21) may be provided to reduce the electrical length difference between the microstrip lines 1111.

FIG. 15 is a schematic structural diagram of a first bend section according to some embodiments of the present disclosure.

In some embodiments, at least one first bend section 1111-1 is included in the arc-shaped structure of the at least one microstrip line 1111, as illustrated in FIG. 15.

The first bend section 1111-1 is a location in the microstrip line 1111 where the curvature is not continuous. It will be appreciated that by providing the first bend section 1111-1, the circumference (or arc length) of the microstrip line 1111 may be increased so that the difference in the length of the electrical paths in an individual microstrip line 1111 is less than or equal to a preset threshold.

In some embodiments, as shown in FIG.15, the microstrip line 1111 may include one or more first bend sections 1111-1, and a count of the one or more first bend sections 1111-1 may be determined based on an electrical length difference of each microstrip line 1111 and a preset threshold. The plurality of first bend sections 1111-1 are uniformly distributed along the circumferential direction of the microstrip line 1111.

It is worth noting that if a bending angle of the first bend section 1111-1 is too large, it will affect the quality of data transmission; if the bending angle of the first bend section 1111-1 is too small, it cannot increase the circumferential length of the microstrip line 1111, so that the electrical length difference among the individual microstrip lines 1111 is less than or equal to the preset threshold. Accordingly, in some embodiments, the bending angle of the first bend section 1111-1 may be within 170° to 180°. For example, the first bend section 1111-1 has a bending angle of 175°.

In some embodiments of the present disclosure, by arranging one or more first bend sections in the arc-shaped structures of the microstrip lines, the total electrical lengths in the plurality of microstrip lines can be made consistent in the case where the plurality of microstrip lines have different radii.

In some embodiments, the end face transmitting antenna 111 includes at least two microstrip lines 1111. Each microstrip line 1111 is connected end-to-end via the transition plate 1111-2 to form the arc-shaped end face transmitting antenna 111. Herein, by the provision of the transition plate 1111-2, the electrical lengths of the microstrip lines 1111 distributed along the radial direction may be supplemented, so that each end face transmitting antenna 111 has equal electrical lengths, thereby ensuring the information transmission quality of the end face transmitting antenna 111.

In some embodiments, at least one microstrip line includes a compensation trace segment connected via an impedance-matching transition. The compensation trace segment refers to a section of microstrip line that is lengthened or reshaped in the microstrip line to compensate for a deviation in electrical length, phase, or impedance. For example, the compensation trace segment includes the first bend section or the microstrip line segment. The impedance-matching transition refers to a gradually changing structure in the microstrip line configured to smoothly transition a characteristic impedance of the compensation trace segment to a characteristic impedance of an adjacent microstrip line (or a load connected end-to-end), thereby eliminating reflections and reducing standing waves. In some embodiments, the impedance-matching transition includes a transition plate.

In some embodiments, as shown in FIG. 14, the microstrip line 1111 includes a transition plate 1111-2 and the microstrip line segment 1111-3. The microstrip line segment 1111-3 is connected to the transition plate 1111-2.

The transition plate 1111-2 is configured to connect the microstrip line segment 1111-3. As shown in FIG. 14, the two microstrip line segments 1111-3 may be connected end-to-end by the two transition plates 1111-2 to form an end face transmitting antenna 111 with a circular arc-like structure.

In some embodiments, the transition plate 1111-2 is connected to the disk where the end face transmitting antenna 111 is located by welding, bolted connection, or other manners. The microstrip line 1111 includes one or more transition plates 1111-2, and the plurality of transition plates 1111-2 are determined based on the electrical length difference of each of the microstrip lines 1111 and a preset threshold. The plurality of transition plates 1111-2 are uniformly distributed along the circumference of the microstrip line 1111.

In some embodiments, the transition plate 1111-2 is configured to address unequal electrical lengths and impedance mismatches in the microstrip line 1111. For example, the compensation wiring 1111-21 is provided on the transition plate 1111-2. For this section, refer to FIG 10 and its related description.. As another example, the microstrip line segment 1111-3 is connected to different positions on the transition plate 1111-2. For details, refer to FIG. 9 and its corresponding descriptions.

It should be noted that the material of the transition plate 1111-2 is not limited, and it is sufficient to be able to be configured for connecting the microstrip line 1111 to form a current path. For example, the material of the transition plate 1111-2 may include a conductive material such as copper, iron, or the like.

The microstrip line segment 1111-3 is a separated electrical path line segment divided by the microstrip lines 1111. In some embodiments, the microstrip line 1111 includes one or more microstrip line segments 1111-3, and a count of the one or more microstrip line segments 1111-3 may be determined based on actual needs. The plurality of microstrip line segments 1111-3 are uniformly distributed along a circumference of the microstrip line 1111.

In some embodiments, when the microstrip line 1111 includes only one microstrip line segment 1111-3, the transition plate 1111-2 is also one, and ends of the microstrip line segments 1111-3 are connected to ends of the transition plate 1111-2. See FIG. 8 and its related descriptions for a description of this section. When the microstrip line 1111 includes a plurality of microstrip line segments 1111-3, the adjacent microstrip line segments 1111-3 may be connected end-to-end via different transition plates 1111-2, and collectively form an arc-shaped microstrip line 1111. For details on this part, refer to the relevant descriptions below. Methods of connecting the microstrip line segment 1111-3 to the transition plate 1111-2 include, but are not limited to, soldering, snap-fitting, or the like.

It is worth noting that the plurality of microstrip line segments 1111-3 of the plurality of microstrip lines 1111 may be the same or different. Additionally, for ease of installation, the microstrip line segments 1111-3 of the plurality of microstrip lines 1111 may be connected to the same transition plate 1111-2. For ease of individual disassembly, assembly, and routine maintenance, the plurality of microstrip line segments 1111-3 of the plurality of microstrip lines 1111 are separately connected to different transition plates 1111-2 to collectively form the arc-shaped end face transmitting antenna 111.

Merely by way of example, as shown in FIG. 14, for the microstrip line 1111 with radius R, the microstrip line 1111 is divided into two microstrip line segments 1111-3. The two microstrip line segments 1111-3 are uniformly distributed along the circumferential direction of the disk (e.g., the first stationary disk 21 or the first rotary disk 31). The two microstrip line segments 1111-3 are connected by two transition plates 1111-2, and collectively form the arc-shaped microstrip line 1111. Similarly, the microstrip line 1111 with radius r is divided into two microstrip line segments 1111-3 that are uniformly distributed along the circumferential direction of the disk. The two microstrip line segments 1111-3 are connected by the same two transition plates 1111-2.

In some embodiments of this disclosure, the transition plate may supplement the electrical lengths of radially distributed microstrip lines to ensure that the end face transmitting antennas have identical electrical lengths, thereby guaranteeing the information transmission quality of the end face transmitting antenna.

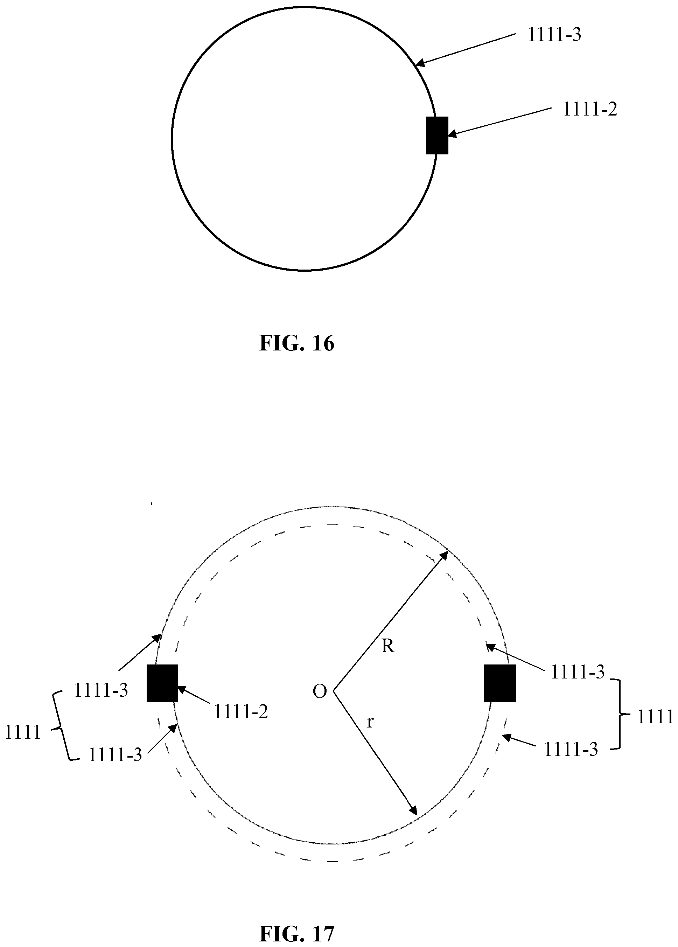

FIG. 16 is a schematic structural diagram of a transition plate and a microstrip line segment according to some embodiments of the present disclosure.

In some embodiments, the microstrip line includes a single impedance-matching transition connected end-to-end with a single microstrip segment.

In some embodiments, as shown in FIG. 16, the microstrip line 1111 includes the transition plate 1111-2 and the microstrip line segment 1111-3. The microstrip line segment 1111-3 is connected end-to-end via the transition plate 1111-2. It is understandable that the microstrip line segment 1111-3 connected end-to-end via the transition plate 1111-2 can form an arc-shaped end face transmitting antenna 111.

In some embodiments of the present disclosure, the microstrip line includes only a transition plate with one microstrip line segment, which can be easily installed and reduces the complexity of operation.

In some embodiments, the microstrip line includes at least two impedance-matching transitions and at least two microstrip segments. Adjacent microstrip segments are interconnected through one of the impedance-matching transitions.

n some embodiments, the microstrip line 1111 includes at least two transition plates 1111-2 and at least two microstrip line segments 1111-3, and adjacent microstrip line segments 1111-3 among the at least two microstrip line segments 1111-3 are connected via a transition plate 1111-2 of the at least two transition plates 1111-2.

The adjacent microstrip line segments 1111-3 refer to the two microstrip line segments 1111-3 that have a closest distance between ends of line bodies in the circumferential direction of the disk.

Merely by way of example, as shown in FIG. 14, for the microstrip line 1111 with radius R, the microstrip line 1111 is divided into two microstrip line segments 1111-3. Both ends of the two microstrip line segments 1111-3 are respectively connected via two transition plates 1111-2, and collectively form the arc-shaped microstrip line 1111.

In some embodiments of the present disclosure, by arranging the plurality of microstrip line segments, it is convenient to replace a certain microstrip line segment individually in an event of a subsequent line failure, which saves resources and facilitates routine maintenance.

In some embodiments, the at least two microstrip line segments 1111-3 have different radii.

FIG. 17 is a schematic diagram illustrating a structure of two microstrip line segments with different radii according to some embodiments of the present disclosure;

Merely by way of example, as shown in FIG. 17, the end face transmitting antenna 111 includes two segments of microstrip lines 1111. One microstrip line 1111 is represented by a dashed line, and the other microstrip line 1111 is represented by a solid line. Each segment of the microstrip line 1111 is divided into two microstrip line segments 1111-3, and the two microstrip line segments 1111-3 have different radii. One of the microstrip line segments 1111-3 has a radius R, the other microstrip line segment 1111-3 has a radius r, and their centers are both point O.

In some embodiments of the present disclosure, connecting microstrip line segments of different radii via transition plates may make the electrical length in any two microstrip line segments less than or equal to a preset threshold, thereby ensuring the information transmission quality of the end-face transmitting antenna.

It is understandable that in actual design, due to conditional constraints, it is not possible to form the plurality of microstrip lines 1111 of equal length at one time. Therefore, by forming microstrip lines 1111 through sequentially connecting the plurality of microstrip line segments 1111-3, and performing length compensation between each pair of the adjacent microstrip line segments 1111-3, it is possible to make the electrical lengths among the plurality of microstrip lines 1111 equal or similar.

FIG. 18 is a schematic diagram illustrating a structure of a compensation wiring disposed in a transition plate according to some embodiments of the present disclosure;

In some embodiments, the at least two microstrip segments have identical radii. Each impedance-matching transition includes a compensation wiring. An electrical path of the microstrip line includes the microstrip segment and the compensation wiring.

In some embodiments, as shown in FIG. 18, at least two microstrip line segments 1111-3 have the same radius. The transition plate 1111-2 is provided with the compensation wiring 1111-21, and the microstrip line segments 1111-3 are connected to the compensation wiring 1111-21. For different microstrip lines 1111, for each microstrip line 1111 among the at least two microstrip line segments 1111-3 with the same radius, the electrical path in the microstrip line 1111 consists of the microstrip line segments 1111-3 and the compensation wirings 1111-21 connected to the microstrip line segments 1111-3.

The compensation wiring 1111-21 refers to a wire that is configured to compensate for an electrical length difference between any two of the plurality of microstrip lines 1111. In some embodiments, the compensation wiring 1111-21 may be connected to the microstrip line end 1111-3 by soldering (e.g., solder joints 1111-24), plugging, or the like.

In some embodiments, a termination resistor 1111-22 is also provided on the transition plate 1111-2 at a tail end of the microstrip line segment 1111-3. For example, as shown in FIG. 18, the microstrip line segment 1111-3 includes two compensation wirings 1111-21, and each of the two compensation wirings 1111-21 is provided with the termination resistor 1111-22, and the termination resistor 1111-22 is connected to the compensation wirings 1111-21 and a reference GND 1111-23.

In some embodiments, the termination resistor 1111-22 also compensates for the electrical length of any two microstrip lines 1111. For example, the termination resistor 1111-22 (also referred to as the first termination resistor) corresponding to the microstrip line 1111 of the outer ring has a larger dimension along the circumferential direction compared with the termination resistor 1111-22 (also referred to as the second termination resistor) corresponding to the microstrip lines 1111 of an inner ring, thereby reducing the difference in electrical lengths between the microstrip lines 1111 of the inner ring and the outer ring.

It should be noted that when the end face transmitting antenna 111 is an arc antenna disposed on the end face of the first rotary disk 31 facing the first stationary disk 21, the specific structure of the end face transmitting antenna 111, the positional relationship between the end face transmitting antenna 111 and the end face receiving antenna 112, and the length compensation of the end face transmitting antenna 111 are all the same in principle as those of the end face transmitting antenna 111.

FIG. 19 is a schematic diagram illustrating a structure of a second bend section according to some embodiments of the present disclosure.

In some embodiments, the compensation wiring 1111-21 includes at least one second bend section 1111-211, as shown in FIG. 19.

The second bend section 1111-211 is configured to increase a line length of the compensation wiring 1111-21. In some embodiments, a count of the second bend section 1111-211 is one or more, and the count of the one or more is determined based on the electrical length difference between any two of the plurality of microstrip lines 1111 and a predetermined threshold.

In some embodiments of the present disclosure, the second bend section may increase a trace length of the compensation wiring, thereby ensuring that the differential pairs of the end face transmitting antenna 111 are routed with equal lengths to guarantee signal transmission quality.

Merely by way of example, as shown in FIG. 14 and FIG. 18, the end face transmitting antenna 111 includes the first microstrip line 1111a and the second microstrip line 1111b spaced apart in the radial direction of the first stationary disk 21. The first microstrip line 1111a includes two first microstrip line segments 1111-3a. The second microstrip line 1111b includes two second microstrip line segments 1111-3b. The first stationary disk 21 is provided with the transition plate 1111-2, and the transition plate 1111-2 is provided with two spaced-apart first compensation wiring 1111-21a and the second compensation wiring 1111-21b. The first compensation wiring 1111-21a and the second compensation wiring 1111-21b are respectively connected to the two first microstrip line segments 1111-3a of the first microstrip line 1111a and the two second microstrip line segments 1111-3b of the second microstrip line 1111b, so that the total length of the first microstrip line 1111a and the first compensation wiring 1111-21a is equal to the total length of the second microstrip line 1111b and the second compensation wiring 1111-21b. Merely by way of example, the length of the first microstrip line 1111a is L1, the length of the second microstrip line 1111b is 11, the length of the first compensation wiring 1111-21a is l2, and the length of the second compensation wiring 1111-21b is L2, and thus the electrical length (e.g., L1 + l2) of the first microstrip line 1111a is equal to the electrical length (e.g., 11 + L2) of the second microstrip line 1111b.

Since the present solution adopts end-face data transmission, the first microstrip line and the second microstrip line in the differential pairs of the transmitting antenna have inconsistent inner diameters, resulting in that the end face transmitting antenna cannot achieve equal length and impedance matching through its own design. Therefore, in some embodiments of the present disclosure, the transition plate is connected to the first microstrip line and the second microstrip line, the transition plate is provided with the first compensation wiring and the second compensation wiring, and the electrical length of the first microstrip line together with the first compensation wiring is made equal to that of the second microstrip line together with the second compensation wiring, thereby ensuring that the differential pairs of the end face transmitting antenna are routed with equal lengths and guaranteeing signal transmission quality.

In some other embodiments, the data and/or signals can also be calibrated or compensate through software, thereby improving the quality of data transmission and/or signal transmission.

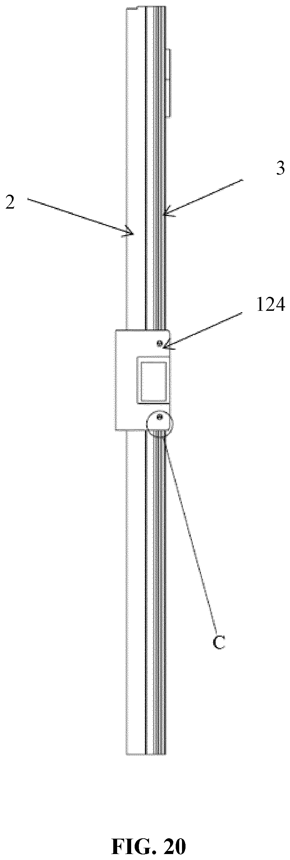

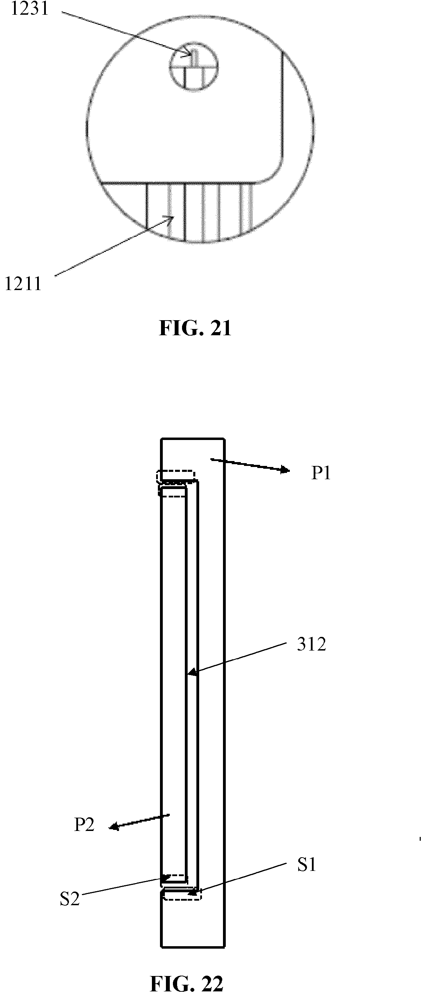

FIG. 20 is a schematic diagram illustrating a structure of a peripheral transceiver according to some embodiments of the present disclosure; FIG. 21 is a schematic diagram illustrating an enlarged view of portion C in FIG. 8.

In some embodiments, the rotary assembly further includes at least one peripheral transceiver. The at least one peripheral transceiver includes a peripheral transmitting antenna disposed on a circumferential surface of one of the stator disk or rotor disk, and a peripheral receiving antenna disposed on the other of the stator disk or rotor disk. The peripheral transmitting antenna and peripheral receiving antenna are radially opposed.

In some embodiments, as shown in FIG. 1, FIG. 20, and FIG. 21, the transceiver 1 further includes at least one peripheral transceiver 12. The peripheral transceiver 12 includes a peripheral transmitting antenna 121 and a peripheral receiving antenna 122. The peripheral transmitting antenna 121 is disposed on the peripheral surface of one of the first rotary component 3 and the first stationary component 2, and the peripheral receiving antenna 122 is disposed on the other of the first rotary component 3 and the first stationary component 2. The peripheral transmitting antenna 121 and the peripheral receiving antenna 122 are coupled to each other.

The peripheral transceiver 12 refers to a transceiver disposed on the peripheral surface of the first stationary component 2 and the first rotary component 3. In some embodiments, the transceiver 1 includes a plurality of peripheral transceivers 12. The plurality of peripheral transceivers 12 are determined in a manner similar to the manner in which the plurality of end-face transceivers 11 are determined, as may be found in the relevant descriptions in FIG.1 - FIG.7.

The peripheral transmitting antenna 121 is a transmitting antenna provided on the peripheral surface of the first stationary component 2 and/or the first rotary component 3. The peripheral receiving antenna 122 refers to a receiving antenna for coupling with the peripheral transmitting antenna 121 in correspondence with each other. It should be noted that the peripheral receiving antenna 122 is not limited to being provided on an end face of the first stationary component 2 and/or the first rotary component 3.

It is understandable that the dimensions, shapes, and structures of the peripheral transmitting antenna 121 and the peripheral receiving antenna 122 are similar to those of the end face transmitting antenna 111 and the end face receiving antenna 112; for details, reference may be made to the relevant descriptions in FIGs. 1-13.

In some embodiments, the peripheral transmitting antenna 121 and the peripheral receiving antenna 122 form capacitive coupling to transmit the control data and/or the image data in a manner similar to that in which the end face transmitting antenna 111 and the end face receiving antenna 112 form capacitive coupling to transmit control data and/or image data; for details, reference may be made to the relevant descriptions in FIG. 1 - FIG. 7.

In some embodiments, referring to FIG. 1 - FIG. 4 and FIG. 20 - FIG. 21, the peripheral transceiver 12 further includes a peripheral surface transmitting plate 124 and a peripheral surface receiving plate 123. The peripheral surface transmitting plate 124 is disposed on the first rotary disk 31. The peripheral transmitting antenna 121 is disposed on the peripheral surface transmitting plate 124 and communicatively connected to the peripheral surface transmitting plate 124. The peripheral surface receiving plate 123 is disposed on the first stationary disk 21, and the peripheral receiving antenna 122 is disposed on the peripheral surface receiving plate 123 and communicatively connected to the peripheral surface receiving plate 123. It is worth noting that the construction of the peripheral surface transmitting plate 124 and the peripheral surface receiving plate 123 is similar to that of the end face transmitting plate 114 and the end face receiving plate 113.

In some embodiments, the peripheral surface receiving plate 123 extends from an outer peripheral surface of the first stationary disk 21 to the outside of the circumferential surface of the first rotary disk 31, such that the peripheral receiving antenna 122 is located on the outer circumference of the peripheral transmitting antenna 121, and the peripheral receiving antenna 122 and the peripheral transmitting antenna 121 are spaced apart along the radial direction of the first stationary disk 21.

In some embodiments, refer to FIG. 20 - FIG. 21, the peripheral surface receiving plate 123 is provided with the peripheral surface alignment mark 1231. The peripheral surface alignment mark 1231 serves to assist in centering the peripheral transmitting antenna 121 and the peripheral receiving antenna 122 along the axial direction of the first stationary disk 21, thereby ensuring the stability of signal transmission between the peripheral transmitting antenna 121 and the peripheral receiving antenna 122. The axial alignment refers to a setting of a center surface of the peripheral transmitting antenna 121 overlapping with a center surface of the peripheral receiving antenna 122 along the axis of the first stationary disk 21.

In this embodiment, since the peripheral transmitting antenna 121 is disposed on the peripheral surface of the first rotary disk 31, the two peripheral surface microstrip lines 1211 of the peripheral transmitting antenna 121 have the same length, thereby eliminating the need for length compensation. Instead, only impedance control of the peripheral transmitting antenna 121 is required.

In some embodiments, the peripheral transmitting antenna 121 is disposed on any position on the first stationary component 2 and/or the first rotary component 3 that meets the signal transmission requirements. That is, the peripheral receiving antenna 122 is disposed on any unobstructed position of the disk on which the peripheral transmitting antenna 121 is not disposed. That is to say, the peripheral receiving antenna 122 may be disposed on the peripheral surface of the disk, or on the end surface of the disk, or on other positions capable of being correspondingly coupled to the peripheral transmitting antenna 121. For a description of the unobstructed position, see the relevant description in FIGs.1 -7.

In some embodiments of the present disclosure, by disposing the plurality of different transceivers, the requirements of multi-channel data and high-capacity data transmission can be met.

FIG. 22 is a schematic diagram illustrating a structure of installing a first rotary disk and a first stationary disk according to some embodiments of the present disclosure.

In some embodiments, as shown in FIG. 22, the first rotary disk 31 includes a notch 312. A shape of the notch 312 matches the shape of the first stationary disk 21. The first stationary disk 21 is rotatably disposed within the notch 312. The peripheral transmitting antenna 121 is disposed on the peripheral surface of the first stationary disk 21 or the peripheral surface of the notch 312 in the first rotary disk 31, and the peripheral receiving antenna 122 is arranged on the other of the first rotary disk 31 and the first stationary disk 21. Alternatively, the first stationary disk 21 includes a notch 312. The shape of the notch 312 matches the shape of the first rotary disk 31, and the first rotary disk 31 is rotatably disposed within the notch 312. The peripheral transmitting antenna 121 is disposed on the peripheral surface of the first rotary disk 31 or the peripheral surface of the notch 312 in the first stationary disk 21, and the peripheral receiving antenna 122 is disposed on the other of the first rotary disk 31 and the first stationary disk 21.

The notch 312 is a groove body that opens on the first stationary disk 21 or the first rotary disk 31. For example, the notch 312 may be open on one of an end face of the first stationary disk 21 proximate to the first rotary disk 31, or the first rotary disk 31 proximate to an end face of the first stationary disk 21.

In some embodiments, the shape of the notch 312 matches that of the disk without a notch to accommodate the disk without the notch and reserve an accommodation space in the circumferential direction to accommodate the peripheral transmitting antenna 121 and the peripheral receiving antenna 122.

Merely by way of example, as shown in FIG. 22, taking the notch 312 opened on the end face of the first stationary disk 21 close to the first rotary disk 31 as an example, the shape and a depth of the notch 312 match the shape and a thickness of the first rotary disk 31 respectively, and the radius of the notch 312 is greater than that of the first rotary disk 31 to form an accommodation space at the edge of the notch 312.

In some embodiments, the axes of the notch 312, the first rotary disk 31, and the first stationary disk 21 are coincident, a gap exists at the end face between the first rotary disk 31 and the first stationary disk 21, and the first rotary disk 31 rotates contactlessly relative to the first stationary disk 21.

It is understandable that, besides the planar end faces between the first rotary disk 31 and the first stationary disk 21 shown in FIG. 22, the end faces between the first rotary disk 31 and the first stationary disk 21 may also be in other shapes, such as curved surfaces or stepped surfaces.

Merely by way of example, as shown in FIG. 14, one of the first rotary disk 31 and the first stationary disk 21 is denoted as P1, and the other is denoted as P2. Mounting gaps S1 and S2 are respectively provided on P1 and P2. One of the peripheral transmitting antenna 121 and the peripheral receiving antenna 122 is disposed within the mounting gap S1, and the other may be disposed within the mounting gap S2.

In some embodiments of the present disclosure, providing a notch can, on one hand, reduce the axial dimension of a rotary assembly. On the other hand, it can avoid unstable signal transmission caused by excessive distance between the peripheral surface transmitting antenna and the peripheral surface receiving antenna.

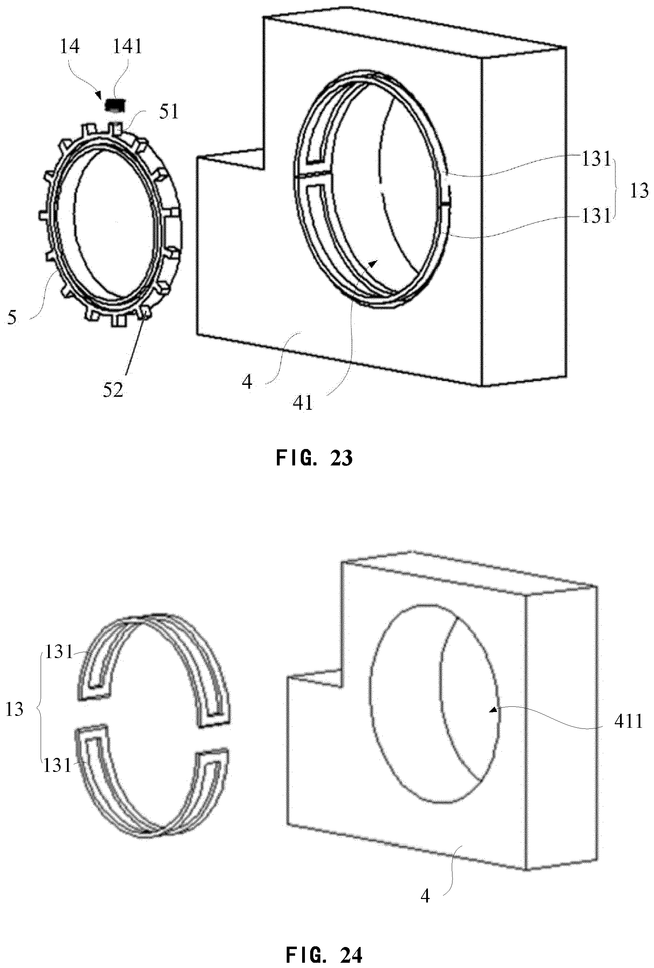

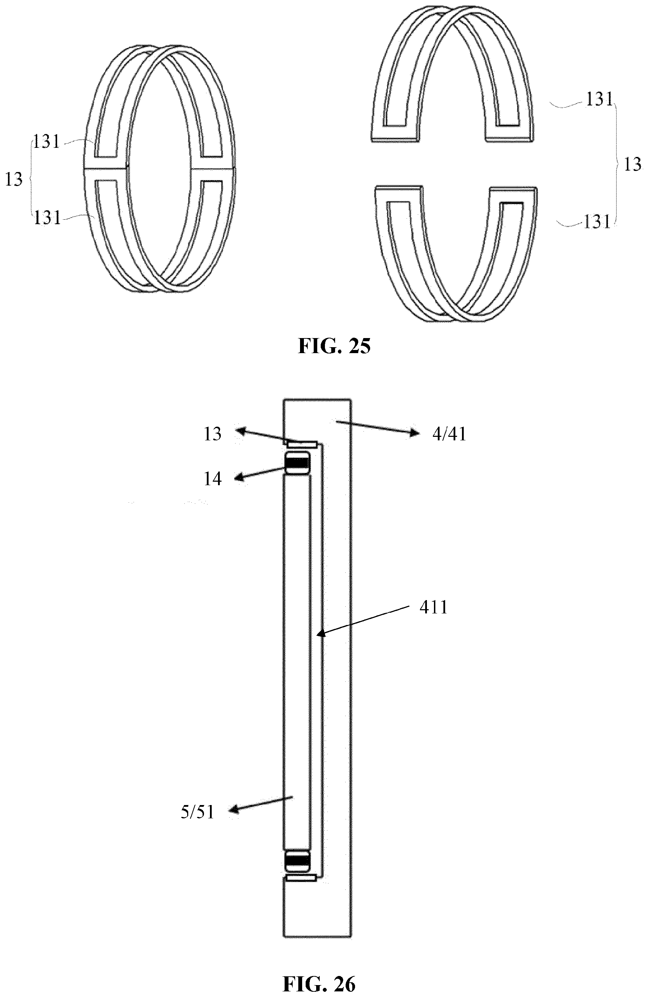

FIG. 23 is a schematic diagram illustrating an exploded structure of the rotary assembly according to some embodiments of the present disclosure; FIG. 24 is a schematic diagram illustrating an exploded structure of a first wound conductor array and a secondary stator component according to some embodiments of the present disclosure; FIG. 25 is a schematic diagram illustrating a three-dimensional structure of modular segments when interconnected and separated according to some embodiments of the present disclosure.

In some embodiments, the rotary assembly further includes a secondary stator component, a secondary rotor, a first wound conductor array and a second wound conductor. The secondary rotor component is rotationally cooperated with the secondary stator component. The first wound conductor array is disposed on the secondary stator component. The second wound conductor disposed on the secondary rotor component. The first wound conductor array comprises modular segments configured for independent parallel operation. In some embodiments, the first wound conductor array and the second wound conductor array are components of the transceiver 1.

In some embodiments, as shown in FIG. 23 - FIG. 25, the rotary assembly further includes a secondary stator component 4 and a secondary rotor component 5 rotatably engaged with the secondary stator component 4. The transceiver 1 further includes the first wound conductor array 13 and the second wound conductor array 14. The first wound conductor array 13 is disposed on the secondary stator component 4, and the first wound conductor array 13 includes modular segments 131 distributed along the circumferential direction of the secondary stator component 4, with at least some of the modular segments 131 connected in parallel to an external transmission electrical path. The second wound conductor array 14 is disposed on the secondary rotor component 5 and coupled with the first wound conductor array 13. Either one of the first wound conductor array 13 and the second wound conductor array 14 is a power transmitting coil, and the other is a power receiving coil.

The secondary stator component 4 is a component of the rotary assembly for securing and supporting the secondary rotor component 5. For example, the secondary stator component 4 comprises a motor stationary member or the like. In some embodiments, the secondary stator component 4 provides a stabilizing magnetic field or mechanical support.

The secondary rotor component 5 is a part of the rotary assembly that rotates about a central axis. For example, the secondary rotor component 5 includes a motor rotating member, etc. In some embodiments, the secondary rotor component 5 transmits energy by rotation and generates a dynamic magnetic field.

In some embodiments, the secondary rotor component 5 is provided in correspondence with the secondary stator component 4. For example, the secondary rotor component 5 is provided coaxially opposite the secondary stator component 4. For another example, the secondary rotor component 5 is provided in the secondary stator component 4, or the secondary stator component 4 is provided in the secondary rotor component 5.

In some embodiments, the secondary stator component 4 is provided with the at least one power transmitting coil. Each power transmitting coil includes a corresponding base structure and a coil, and multi-turn coils are wound on the base structure to form a power receiving coil.

The first wound conductor array 13 and the second wound conductor array 14 are configured to realize power and/or signal transmission. In some embodiments, the first wound conductor array 13 is disposed inside the secondary stator component 4, and the second wound conductor array 14 is disposed on the peripheral surface of the secondary rotor component 5.

A modular segment 131 refers to a separate electrical circuit in the first wound conductor array 13. In some embodiments, the modular segment 131 may be saddle-shaped. A count of the modular segments 131 may be one or more, and the count of the modular segments 131 is determined according to the size of the peripheral surface (e.g., the mounting aperture 411) of the secondary stator component 4. The plurality of modular segments 131 are evenly distributed along the circumference of the first wound conductor array 13. Optimally, a count of the plurality of modular segments 131 is two, and the two modular segments 131 are circularly shaped, and each encloses to form a shape that adheres to the peripheral surface of the secondary stator component 4. See the related description below for a description of the mounting aperture 411.

In some embodiments, the plurality of modular segments 131 are connected in parallel to the external transmission circuit, thereby transmitting power or signals in parallel.

At least some of the modular segments 131 being connected in parallel to the external transmission circuitry means that at least some of the modular segments 131 are capable of transmitting power or signals in parallel. That is, the modular segments 131 in this portion transmit power or signals independently of each other without interference. When some of the modular segments 131 in this portion malfunction and cannot operate normally, the other modular segments 131 can still transmit power or signals normally.

Taking FIG. 23 - FIG. 25 as an example, the count of modular segments 131 is two. The two modular segments 131 are distributed along the circumferential direction of the secondary stator component 4. The two modular segments 131 are connected in parallel to the external transmission circuit, so that the two modular segments 131 may transmit power or signals in parallel. It is understandable that the two modular segments 131 transmit power or signals independently of each other. When one of the two modular segments 131 malfunctions (e.g., in case of a disconnection) and cannot transmit power or signals normally, the other modular segment 131 is unaffected. For example, the modular segment 131 may continue to transmit power or signals. The external transmission circuit refers to a power supply circuit provided outside of the rotary assembly.

In some embodiments, the second wound conductor array 14 is coupled in parallel with each modular segment 131 of the first wound conductor array 13. Moreover, when the secondary rotor component 5 rotates relative to the secondary stator component 4, power or signals may be transmitted between the second wound conductor array 14 on the secondary rotor component 5 and the first wound conductor array 13 on the secondary stator component 4.

In some embodiments, the first wound conductor array 13 is a power transmitting coil, and the second wound conductor array 14 is a power receiving coil. The second wound conductor array 14 is power-coupled to the first wound conductor array 13 to form a power-coupled loop, and the first wound conductor array 13 may transmit power to the second wound conductor array 14. Alternatively, the first wound conductor array 13 serves as a power-receiving coil, and the second wound conductor array 14 serves as a power-transmitting coil. The second wound conductor array 14 is power-coupled to the first wound conductor array 13 to form a power-coupled loop, and the second wound conductor array 14 may transmit power to the first wound conductor array 13.

In other embodiments, the first wound conductor array 13 is a signal transmitting coil, and the second wound conductor array 14 is a signal receiving coil. The second wound conductor array 14 is signal-coupled to the first wound conductor array 13 to form a signal coupling loop, and the first wound conductor array 13 may transmit signals to the second wound conductor array 14. Alternatively, the first wound conductor array 13 acts as a signal-receiving coil, while the second wound conductor array 14 acts as a signal-transmitting coil. The second wound conductor array 14 is signal-coupled to the first wound conductor array 13 to form a signal-coupling loop, enabling the second wound conductor array 14 to transmit signals to the first wound conductor array 13.

In some embodiments of the present disclosure, by providing the secondary stator component and the secondary rotor component, the transmitting coil can continuously transmit power or signals to the receiving coil while the secondary rotor component rotates relative to the secondary stator component. By connecting at least some of the modular segments of the first wound conductor array in parallel to the external transmission circuit, these modular segments can transmit power or signals in parallel. Therefore, when any one of these modular segments malfunctions and fails to transmit power or signals normally, the remaining modular segments can still continue to transmit power or signals, ensuring the normal operation of the slip ring and thus improving the stability and reliability of the mechanical equipment.

In some embodiments, the modular segments are detachably mounted on the secondary stator component.

The modular segment 131 may be independently and detachably arranged on the secondary stator component 4 in a variety of ways. In some embodiments, the modular segment 131 may be snapped onto the secondary stator component 4. For example, by providing the plurality of spaced-apart slots on the peripheral surface of the secondary stator component 4, the modular segment 131 is snapped into the corresponding slot(s) by an interference fit. In some embodiments, the modular segment 131 may be bonded to the secondary stator component 4. For example, the modular segment 131 is glued to a peripheral surface of the secondary stator component 4 by a gel. In some embodiments, the modular segment 131 may also be snapped to the secondary stator component 4. For example, the plurality of spaced-apart snap hooks are provided on the peripheral surface of the secondary stator component 4. The snap grooves are provided on each modular segment 131. The modular segments 131 are placed on the peripheral surface of the secondary stator component 4, and then the snap hooks are snapped into the corresponding snap grooves. In some embodiments, the modular segment 131 may also be mounted by using fasteners such as bolts, screws, or the like, and this disclosure does not limit the mounting method thereof.

It should be appreciated that in other embodiments, it is also possible to splice all of the modular segments 131 together before assembling them to the secondary stator component 4.

In some embodiments of the present disclosure, by independently and detachably mounting at least some of the modular segments on the secondary stator component, when some of the modular segments malfunction, only those modular segments need to be disassembled and replaced. There is no need to disassemble or replace the entire first wound conductor array, thus helping to save costs and reduce labor intensity.

In some embodiments, two adjacent modular segments 131 are insulated from each other. For example, an insulator is provided between two adjacent modular segments 131. Optionally, the insulator may be made of plastic, rubber, ceramic, glass, polymer material, etc., and the insulator is configured to electrically insulate between the two adjacent modular segments 131.

It is understandable that by providing an insulator between the two adjacent modular segments 131, the insulator insulates the two adjacent modular segments 131 from each other, effectively preventing electrical interference therebetween and ensuring the stability and reliability of the power or signals transmitted by each modular segment 131.

In some embodiments, each of the modular segments 131 is provided with a positive connector and a negative connector, and each of the modular segments 131 may be electrically connected to the external transmission circuit and/or the external detection device via the positive connector and/or the negative connector. Optionally, the external transmission circuit is an inverter circuit, and the modular segments 131 are connected to the inverter circuit through their respective positive connectors and negative connectors to form a parallel power or signal transmission relationship. Alternatively, the external detection device may measure impedances of the positive connector and the negative connector. This helps detect whether the circuit of each modular segment 131 is turned on or off. It enables fault diagnosis of the modular segments 131. Thus, the faulty modular segments 131 can be located quickly and accurately. In this way, only the faulty modular segments 131 need to be replaced.

In some embodiments, the secondary stator component defines a mounting aperture. The first wound conductor array is disposed on an inner peripheral wall of the mounting aperture. The secondary rotor component is rotatably disposed within the mounting aperture. The second wound conductor is disposed on an outer peripheral wall of the secondary rotor component.

In some embodiments, as shown in FIG. 23 - FIG. 25, the secondary stator component 4 has a mounting aperture 411. The first wound conductor array 13 is arranged on an inner peripheral wall of the mounting aperture 411. The secondary rotor component 5 is rotatably disposed within the mounting aperture 411. The second wound conductor array 14 is disposed on an outer peripheral wall of the secondary rotor component 5 and is radially coupled to the first wound conductor array 13.

FIG. 26 is a schematic diagram illustrating a structure of installing a secondary rotor component and a secondary stator component according to some embodiments of the present disclosure.

In some embodiments, as shown in FIG. 26, the secondary stator component 4 includes a second stationary disk 41, and the secondary rotor component 5 includes a second rotary disk 51. The second stationary disk 41 has a mounting aperture 411. The second rotary disk 51 is rotatably disposed within the mounting aperture 411. The second wound conductor array 14 is disposed on the outer peripheral wall of the second rotary disk 51.

The second stationary disk 41 is a main structure of the secondary stator component 4. The second rotary disk 51 is a main structure of the secondary rotor component. In some embodiments, the second stationary disk 41 may be the same as or different from the first stationary disk 21 and the second rotary disk 51 may be the same as or different from the first rotary disk 31.

The outer peripheral wall of a structure is a circumferential wall of the structure away from the axis of the secondary stator component 4 or the secondary rotor component 5. The inner peripheral wall of a structure is a circumferential side wall of the structure proximate the axis of the secondary stator component 4 or the secondary rotor component 5.

The mounting aperture 411 is an opening in the second stationary disk 41. In some embodiments, the mounting aperture 411 is configured to mount the secondary stator component 4 and the secondary rotor component 5. The way of mounting the secondary stator component 4 and the secondary rotor component 5 through the mounting aperture 411 is similar to the way of mounting the first stationary component 2 and the first rotary component 3 through the notch 312. For details, refer to the relevant description in FIG. 22, and it will not be repeated here. Similarly, the first wound conductor array 13 and the second wound conductor array 14 may be provided on a peripheral surface between the secondary stator component 4 and the secondary rotor component 5.

In some embodiments of the present disclosure, the size of the rotary assembly can be reduced by providing a mounting aperture to integrate the data transmission with the power transmission.

In some embodiments, since the first wound conductor array 13 is disposed on the inner peripheral wall of the mounting aperture 41 and the second wound conductor array 14 is disposed on the outer peripheral wall of the secondary rotor component 5, when the secondary rotor component 5 is installed within the mounting aperture 41, the first wound conductor array 13 and the second wound conductor array 14 are arranged between the secondary stator component 4 and the secondary rotor component 5 along the radial direction of the mounting aperture 41. This arrangement enables the first wound conductor array 13 and the second wound conductor array 14 to achieve power or signal coupling along the radial direction of the mounting aperture 41.

In some embodiments, the modular segments 131 of the first wound conductor array 13 are distributed along the inner peripheral wall of the mounting aperture 411. Further, all the modular segments 131 are compactly arranged on the inner peripheral wall of the mounting aperture 411 along the circumferential direction of the mounting aperture 411, so that an outer contour size of the first wound conductor array 13 substantially matches a contour of the mounting aperture 411. In this way, when the secondary rotor component 5 drives the second wound conductor array 14 to rotate relative to the secondary stator component 4, the first wound conductor array 13 can continuously and without interruption transmit power or signals to the second wound conductor array 14.

The above technical solution involves opening the mounting aperture 411 on the secondary stator component 4, arranging the first wound conductor array 13 on the inner peripheral wall of the mounting aperture 411, placing the secondary rotor component 5 inside the mounting aperture 411, and disposing the second wound conductor array 14 on the outer peripheral wall of the secondary rotor component 5. This setup arranges the first wound conductor array 13 and the second wound conductor array 14 between the secondary stator component 4 and the secondary rotor component 5 along the radial direction of the mounting aperture 411, achieving radial coupling between the first wound conductor array 13 and the second wound conductor array 14. This helps improve the stability of power or signal transmission between the first wound conductor array 13 and the second wound conductor array 14.

In some embodiments, the first wound conductor array 13 and the second wound conductor array 14 are spaced apart in a radial direction along the mounting aperture 411, and the first wound conductor array 13 and the second wound conductor array 14 are non-contact coupled. For example, in the radial direction of the mounting aperture 411, there is a preset distance between the first wound conductor array 13 and the second wound conductor array 14, and there is wireless power or signal coupling between the first wound conductor array 13 and the second wound conductor array 14. In this way, when the second wound conductor array 14 rotates with the secondary rotor component 5, the second wound conductor array 14 does not contact the first wound conductor array 13, so that wireless transmission of power or signals is performed between the first wound conductor array 13 and the second wound conductor array 14.

In some embodiments, carbon brushes may be disposed between the first wound conductor array 13 and the second wound conductor array 14 along the radial direction of the mounting aperture 411, enabling coupling between the first wound conductor array 13 and the second wound conductor array 14 via the carbon brushes. For example, carbon brushes are disposed on the modular segments 131 of the first wound conductor array 13. When the secondary rotor component 5 drives the second wound conductor array 14 to rotate relative to the secondary stator component 4, the carbon brushes may come into contact with the second wound conductor array 14, achieving contact-based coupling between the first wound conductor array 13 and the second wound conductor array 14.

In some embodiments of the present disclosure, the first wound conductor array 13 and the second wound conductor array 14 achieve contact coupling via carbon brushes. This enables power or signals to be transmitted between the first wound conductor array 13 and the second wound conductor array 14 through the carbon brushes, ensuring good transmission stability.

In some embodiments, referring to FIG. 23 and FIG. 25, the count of modular segments 131 is more than one, and the plurality of modular segments 131 are spliced along the circumferential direction of the secondary stator component 4 to form an annular structure, which is adapted to the mounting aperture 411.

The splicing of the plurality of modular segments 131 along the circumferential direction of the secondary stator component 4 means that, in the circumferential direction of the mounting aperture 411, the ends of two adjacent modular segments 131 are correspondingly arranged and close to each other, and insulation is provided between the two adjacent modular segments 131. The ring structure being adapted to the mounting aperture 411 means: the outer contour dimension of the ring structure is adapted to the dimension of the mounting aperture 411. When the first wound conductor array 13 is mounted to the secondary stator component 4, an outer peripheral wall of the annular structure is fitting with an inner peripheral wall of the mounting aperture 411.

In some embodiments of the present disclosure, splicing the plurality of modular segments 131 into an annular structure along the circumferential direction of the secondary stator component 4, which matches the mounting aperture 411, helps increase the contact area between the first wound conductor array 13 and the inner peripheral wall of the mounting aperture 411. This allows the modular segments 131 to better adhere to the inner peripheral wall of the mounting aperture 411, enhancing the stability of the modular segments 131 mounted on the secondary stator component 4. Meanwhile, it improves the utilization of the space within the mounting aperture 411.

In some embodiments, the mounting aperture 411 is circular, and the each modular segment 131 is curved and adapted to the inner peripheral wall of the mounting aperture 411. For example, the respective modular segments 131 are of the same shape and size, and the respective modular segments 131 are arc-shaped and concentric with the mounting aperture 411. The plurality of modular segments 131 are uniformly distributed along the circumferential direction of the mounting aperture 411 so as to make the annular structure substantially circular, and the annular structure is set at the same center of the circle as the mounting aperture 411.

In some embodiments of the present disclosure, configuring the modular segment 131 into a curved shape matching the inner peripheral wall of the mounting aperture 411 helps increase the contact area between the modular segment 131 and the inner peripheral wall of the mounting aperture 411, enabling the modular segment 131 to adhere more stably to the inner peripheral wall of the mounting aperture 411.

In some embodiments, the modular segment 131 includes a support body and a first wound conductor array body, the support body being curved, and the first wound conductor array body being wound on the support body.

Optionally, the support body is made of a rigid insulating material, the support body is provided with a through opening, and the first wound conductor array body is wound around the through opening on a side of the support body facing the secondary rotor component 5. The plurality of first wound conductor array bodies are a plurality of turns, and the plurality of turns of the first wound conductor array bodies are arranged sequentially in a direction from an inner edge of the support body to an outer edge of the support body.

In some embodiments of the present disclosure, winding the first wound conductor array body around a curved support structure enables the modular segment 131 to maintain a stable curved configuration while facilitating easy assembly and disassembly.

In some embodiments, referring to FIG. 23, the second wound conductor array 14 includes the plurality of second wound conductor array bodies 141, the plurality of second wound conductor array bodies 141 being spaced apart in a circumferential direction along the secondary rotor component 5.

Optionally, the secondary rotor component 5 is circular in shape and is disposed concentrically with the mounting aperture 411, and the plurality of second wound conductor array bodies 141 are disposed at equidistant intervals along the circumferential direction of the secondary rotor component 5.

In some embodiments of the present disclosure, by distributing the plurality of second wound conductor array bodies 141 of the second wound conductor array 14 at equidistant intervals along the circumferential direction of the secondary rotor component 5, when the second wound conductor array 14 rotates with the secondary rotor component 5, continuous and stable power or signal transmission between the first wound conductor array 13 and the second wound conductor array 14 is enabled.

In some embodiments, the plurality of winding posts 52 are disposed on the outer peripheral wall of the secondary rotor component 5. The plurality of winding posts 52 are spaced apart circumferentially along the secondary rotor component 5, and each of the plurality of second wound conductor array bodies 141 is wound around a respective winding post 52. Merely by way of example, the count of the winding posts 52 is equal to the count of the second wound conductor array bodies 141. The plurality of winding posts 52 are evenly arranged along the circumferential direction of the secondary rotor component 5 and extend in the radial direction of the secondary rotor component 5 toward the first wound conductor array 13, and the plurality of second wound conductor array bodies 141 are respectively helically wound one-to-one around the plurality of winding posts 52. The winding posts 52 may be square-rectangular, cylindrical, etc.

In some embodiments of the present disclosure, by winding each of the plurality of second wound conductor array bodies 141 around a respective one of the plurality of winding posts 52, this facilitates fabrication and enables the second wound conductor array 14 to be mounted more stably on the secondary rotor component 5.

In other embodiments, the plurality of second wound conductor array bodies 141 may also be arranged along the circumferential direction of the secondary rotor component 5 on the outer peripheral wall of the secondary rotor component 5 by bonding. Alternatively, a plurality of grooves may be formed on the outer peripheral wall of the secondary rotor component 5, and the plurality of second wound conductor array bodies 141 may be respectively and one-to-one accommodated in the plurality of grooves.

In some embodiments of the present disclosure, by providing a mounting aperture and disposing the second wound conductor array on the outer peripheral wall of the rotating member, radial coupling between the first wound conductor array and the second wound conductor array is achieved, which helps enhance the stability of power or signal transmission between the first wound conductor array and the second wound conductor array.

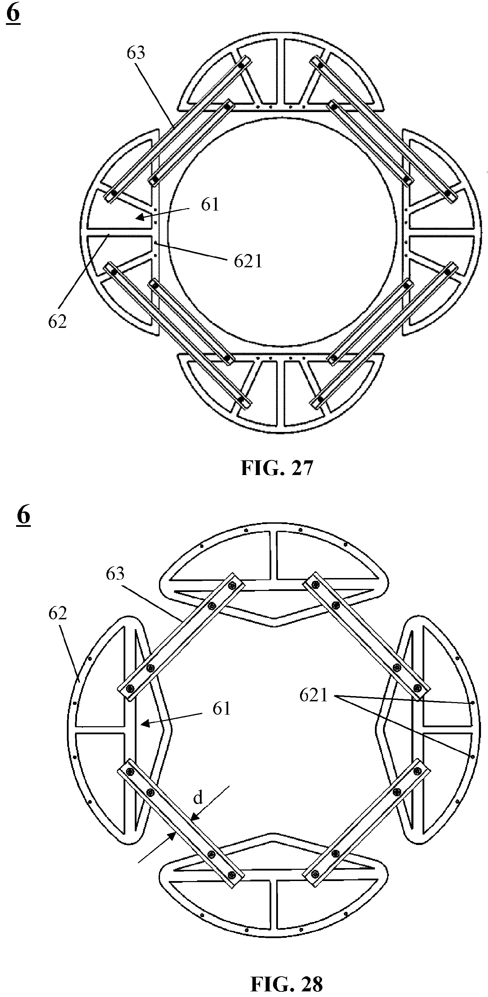

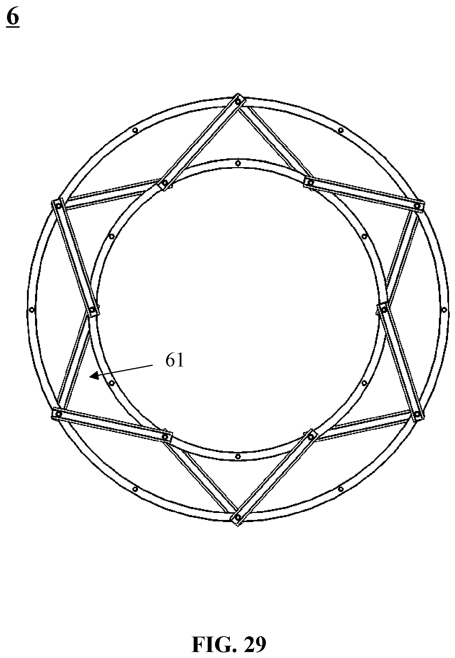

FIG. 27 is a schematic diagram illustrating a cast reinforcement frame according to some embodiments of the present disclosure; FIG. 28 is a schematic diagram illustrating another cast reinforcement frame according to some embodiments of the present disclosure; FIG. 29 is a schematic diagram illustrating yet another cast reinforcement frame according to some embodiments of the present disclosure.

In some embodiments, at least one of the stator disk or the rotor disk includes a cast reinforcement frame embedded therein.

In some embodiments, the cast reinforcement frame 6 is arranged along a circumferential direction of the disk. The disk includes the first rotary disk 31 and/or the first stationary disk 21.

In some embodiments, the disk further includes the second rotary disk 51 and/or the second stationary disk 41. In some embodiments, the cast reinforcement frame 6 is embedded into the disk to increase the stiffness of the disk.

The cast reinforcement frame 6 refers to a frame structure that is configured to reinforce the strength of the disk when cast. In some embodiments, the cast reinforcement frame 6 may be set in a variety of shapes depending on the needs of the disk. FIG.27 - FIG.29 only show a partial structure of the cast reinforcement frame 6, in other embodiments, the cast reinforcement frame 6 may also be of other shapes. The cast reinforcement frame 6 may be made of one or more of non-magnetic, corrosion-resistant, and high-strength materials, such as non-magnetic metal, corrosion-resistant alloys, composite materials, and the like.

In some embodiments, a center of the cast reinforcement frame 6 coincides with the axis of the disk. A centroid of the cast reinforcement frame 6 is hollowed out. The cast reinforcement frame 6 is a centrally symmetric structure, and the cast reinforcement frame 6 is continuous along its circumferential direction, so as to avoid insufficient rigidity at corresponding positions caused by the lack of the cast reinforcement frame in partial circumferential regions.

In some embodiments, the cast reinforcement frame forms a continuous toroidal structure.

In some embodiments, the cast reinforcement frame is arranged along a circumferential direction of the disk.

In some embodiments, the cast reinforcement frame 6 includes a plurality of reinforcing ribs. The plurality of reinforcing ribs are uniformly distributed along the entire circumference of the cast reinforcement frame 6. The plurality of reinforcing ribs are sequentially connected to form a closed loop. This ensures that the poured disk has consistent stiffness.

In some embodiments of the present disclosure, the circumferential uniform distribution of the reinforcing ribs prevents insufficient rigidity at corresponding positions caused by the absence of the cast reinforcement frame in partial circumferential regions.

Merely by way of example, a casting process of the disk may include: designing the cast reinforcement frame 6 based on the shape of the disk; assembling the cast reinforcement frame 6, placing the assembled cast reinforcement frame 6 into a casting mold, and making the centroid of the cast reinforcement frame 6 coincide with the axis of the disk; and casting with a rigid material (e.g., cast iron) until the casting is complete.

In some embodiments of the present disclosure, the cast reinforcement frame is poured into the disk, which can enhance the rigidity of the disk, and there is no need to process reinforcing bars again, which saves the material used and the labor time.

In some embodiments of the present disclosure, by limiting the size of the cast reinforcement frame along the radial direction of the disk, it is possible to avoid damage due to low strength in some areas of the disk (e.g., at the smallest dimension) and to prevent excessive weight used for the radial dimension from being too large. And, by limiting the yield strength of the cast reinforcement frame, the weak areas of bending resistance can be minimized to safeguard the overall strength of the disk.

In some embodiments, a radial span of the cast reinforcement frame occupies 5%-20% of a diameter of the disk.

That is, a ratio of a coverage width of the cast reinforcement frame 6 along the radial direction of the disk to a diameter of the disk is within a range of 0.05 to 0.2.

The coverage width of the cast reinforcement frame 6 along the radial direction of the disk is defined as a difference between a dimension of an outer contour of the cast reinforcement frame 6 and a dimension of an inner contour in a radial direction along the disk. It should be noted that when the inner contour and the outer contour of the cast reinforcement frame 6 are irregular, the coverage width of the cast reinforcement frame 6 in the radial direction along the disk may be a difference between a maximum outer circle and a minimum inner tangent circle of the cast reinforcement frame 6. The centers of both the maximum outer circle and the minimum inner tangent circle coincide with the center of the disk.

In some embodiments, the coverage width of the cast reinforcement frame 6 along the radial direction of the disk includes a width of a hollow portion 61. For more descriptions of the hollow portion 61 may be found in the following descriptions.

In some embodiments, the range of the ratio of the coverage width of the cast reinforcement frame 6 along the radial direction of the disk to the diameter of the disk may also be within a range of 0.05-0.1. For example, the range of the ratio of the coverage width of the cast reinforcement frame 6 along the radial direction of the disk to the diameter of the disk is within a range of 0.1-0.25.

It is understandable that the greater the coverage of the reinforcing ribs in the radial direction, the better the stress performance of the poured disk; however, the weight of the disk is also greater, which will lead to greater power consumption during subsequent rotation.

In some embodiments of the present disclosure, by limiting the ratio of the coverage width of the cast reinforcement frame in the radial direction of the disk to the diameter of the disk, not only can the weight of the disk be reduced to lower power consumption during subsequent rotation, but also the disk can be ensured to have sufficient supporting strength.

It is understandable that the thicker the reinforcing ribs, the rigidity of the reinforcing ribs; however, this will increase the weight of the reinforcing ribs, hence the need for a hollowed design in the reinforcing ribs.

In some embodiments, the cast reinforcement frame includes at least one hollow portion 61. The at least one hollow portion 61 is centrally symmetric about an axis of the disk.

The hollow portion 61 refers to an opening formed between a plurality of reinforcing ribs. In some embodiments, a count of the hollow portion 61 may be one or more, and the specific count may be set according to actual needs.

In some embodiments, the shapes of the plurality of hollow portions 61 may be the same or different. The plurality of hollow portions are uniformly distributed along the circumferential direction of the cast reinforcement frame 6, and with the centroid of the cast reinforcement frame 6 as the center, the plurality of hollow portions present a centrally symmetric distribution.

In some embodiments of the present disclosure, by providing a plurality of hollow portions distributed centrally, the circumferential distribution of the rigidity of the disk can be ensured to be uniform, while the weight of the disk is reduced, which saves electricity when the disk rotates subsequently. In some embodiments, the ratio range of the coverage width of the cast reinforcement frame 6 along the radial direction of the disk to the diameter of the disk may be within a range of 0.05-0.2. Through the aforesaid restrictions, the problems of the supporting force of the disk and the energy consumption during the rotation of the disk can be effectively solved; while reducing the weight of the disk to make the subsequent rotation of the disk more energy-efficient, it ensures the uniformity of the force on the disk and guarantees that the disk has sufficient supporting force.

In some embodiments, the cast enforcement frame includes a plurality of detachably interconnected grid plates 62 and cross-members 63. The grid plates 62 include threaded bores configured for engagement with ejection fixtures during demolding.

A grid plate 62 refers to a hollow plate composed of multiple reinforcing ribs. In some embodiments, as shown in FIGs. 27-28, the grid plate is designed in various structural shapes, such as a fan-shape or a semi-circular shape. Each grid plate is provided with one or more hollow portions 61. In some embodiments, the grid plate 62 is connected by the cross-members 63 to form a continuous ring structure.

A cross-member 63 is detachably connected two adjacent grid plates 62. The cross-members 63 can be detachably connected with the two adjacent grid plates 62 in various manners, such as threaded connection, clamping, etc. In some embodiments, a count and a size (e.g., a length) of the cross-member 63 connecting two adjacent grid plates is determined according to actual requirements. For example, the count of cross-members 63 may be 1 or 2, etc. It is worth noting that the cross-members 63 is also be referred to as the reinforcing ribs mentioned above.

In some embodiments, each grid plate 62 is provided with multiple threaded bores 621. One or more of the threaded bores 621 is configured to connect the cross-member 63 (such as through fasteners to connect with the cross-member 63), and the remaining multiple threaded bores are configured to connect with the ejection fixtures, so as to well demold.

The ejection fixtures refers to components cooperate with some of the threaded bores 621 on the grid plate during demolding to achieve smooth demolding. For example, the ejection fixtures include hangers, etc.

In some embodiments, during the demolding process, the ejection fixtures cooperate with multiple threaded bores 621 on the grid plate to apply uniform pulling force to the cast reinforcing frame structure 6 for demolding, thereby preventing the structure of the disk from deforming.

In some embodiments of this specification, during the pouring and demolding process, by using the ejection fixtures to cooperate with the multiple threaded bores 621 set on the grid plate, to apply uniform pulling force to the reinforcing frame structure, the demolding process is simple, convenient and reliable.

The present disclosure further provides a computed tomography device, which includes a frame and the rotary assembly according to any one of the foregoing embodiments, and the rotary assembly is disposed on the frame. The computed tomography device, through the arrangement of the rotary assembly of the above-mentioned data link, can ensure stable signal transmission and stable operation of the computed tomography device. Moreover, when the rotary component in the rotary assembly rotates relative to the stationary component, the transmitting coil continuously transmits power or signals to the receiving coil. By connecting at least part of the modular segments of the first wound conductor array in parallel to an external transmission circuit, the part of the modular segments can transmit power or signals in parallel. Thus, when any part of the modular segments in this part fails to transmit power or signals normally, the remaining modular segments can still continue to transmit power or signals to maintain the normal operation of the slip ring, which in turn helps improve the stability and reliability of the operation of mechanical equipment.

The basic concepts have been described above, and it is apparent to a person skilled in the art that the above detailed disclosure is intended only as an example and does not constitute a limitation of the present disclosure. While not expressly stated herein, various modifications, improvements, and amendments may be made to this disclosure by those skilled in the art. Those types of modifications, improvements, and amendments are suggested in this disclosure, so those types of modifications, improvements, and amendments remain within the spirit and scope of the exemplary embodiments of this disclosure.

## Claims

1. A rotary assembly, the rotary assembly comprising:
a stator disk;
a rotor disk rotationally cooperated with the stator disk;
at least one transmitting antenna disposed on a mating surface of one of the stator disk or the rotor disk;
at least one receiving antenna disposed on the mating surface of the other of the stator disk or the rotor disk;
wherein the transmitting antenna and the receiving antenna are electromagnetically coupled to maintain signal integrity during relative rotation.

2. The rotary assembly according to claim 1, further comprising:
a first end-face transceiver and a second end-face transceiver;
wherein:
a first transmitting antenna of the first end-face transceiver is disposed on a first mating surface of the rotor disk;
a first receiving antenna of the first end-face transceiver is disposed on the stator disk;
a second transmitting antenna of the second end-face transceiver is disposed on a second mating surface of the stator disk; and
a second receiving antenna of the second end-face transceiver is disposed on the rotor disk.

3. The rotary assembly according to claim 1 or 2, wherein the end face transmitting antenna includes at least two microstrip lines, the at least two microstrip lines are concentric arcuate conductors spaced and concentrically arranged along a radial direction, and a length difference between two electrical paths of any two microstrip lines of the at least two microstrip lines is less than or equal to a preset threshold.

4. The rotary assembly according to claim 3, wherein at least one microstrip line comprises:
a compensation trace segment connected via an impedance-matching transition.

5. The rotary assembly according to claim 4, wherein the microstrip line comprises:
a single impedance-matching transition connected end-to-end with a single microstrip segment.

6. The rotary assembly according to claim 4 or 5, wherein the microstrip line comprises:
at least two impedance-matching transitions and at least two microstrip segments,
adjacent microstrip segments interconnected through one of the at least two impedance-matching transitions.

7. The rotary assembly according to claim 6, wherein:
the at least two microstrip segments have identical radii;
each impedance-matching transition includes a compensation wiring; and
an electrical path of the microstrip line comprises the microstrip segment and the compensation wiring.

8. The rotary assembly according to any one of claims 1-7, further comprising:
at least one peripheral transceiver including:
a peripheral transmitting antenna disposed on a circumferential surface of one of the stator disk or the rotor disk;
a peripheral receiving antenna disposed on the other of the stator disk or the rotor disk;
wherein the peripheral transmitting antenna and the peripheral receiving antenna are radially opposed.

9. The rotary assembly according to any one of claims 1-8, further comprising:
a secondary stator component;
a secondary rotor component rotationally cooperated with the secondary stator component;
a first wound conductor array disposed on the secondary stator component;
a second wound conductor array disposed on the secondary rotor component;
wherein the first wound conductor array comprises modular segments configured for independent parallel operation.

10. The rotary assembly according to claim 9, wherein:
the secondary stator component defines a mounting aperture;
the first wound conductor array is disposed on an inner peripheral wall of the mounting aperture;
the secondary rotor component is rotatably disposed within the mounting aperture; and
the second wound conductor is disposed on an outer peripheral wall of the secondary rotor component.

11. The rotary assembly according to any one of claims 1-10, wherein at least one of the stator disk or the rotor disk comprises:
a cast reinforcement frame embedded therein.

12. The rotary assembly according to claim 11, wherein:
the cast reinforcement frame forms a continuous toroidal structure.

13. The rotary assembly according to claim 11 or claim 12, wherein:
the cast reinforcement frame includes at least one hollow portion; and
the at least one hollow portion is centrally symmetric about an axis of the disk.

14. The rotary assembly according to any one of claims 11-13, wherein:
the cast reinforcement frame comprises a plurality of detachably interconnected grid plates and cross-members;
wherein the grid plates include threaded bores configured for engagement with ejection fixtures during demolding.

15. A computed tomography apparatus comprising:
a gantry; and
a rotary assembly according to any one of claims 1-14 mounted on the gantry.
